(19) Europäisches Patentamt
European Patent Office
Office européen des brevets

(11) EP 4 227 416 A1

(12) EUROPEAN PATENT APPLICATION
published in accordance with Art. 153(4) EPC

(43) Date of publication:
**16.08.2023 Bulletin 2023/33**

(21) Application number: **21877759.7**

(22) Date of filing: **08.10.2021**

(51) International Patent Classification (IPC):
***C12N 15/87*** (2006.01) ***A61K 31/7088*** (2006.01)
***A61K 31/7105*** (2006.01) ***A61K 31/713*** (2006.01)
***A61K 47/61*** (2017.01) ***A61K 48/00*** (2006.01)
***A61P 31/10*** (2006.01) ***C12N 15/113*** (2010.01)

(52) Cooperative Patent Classification (CPC):
**A61K 31/7088; A61K 31/7105; A61K 31/713; A61K 47/61; A61K 48/00; A61P 31/10; C12N 15/113; C12N 15/87**

(86) International application number:
**PCT/JP2021/037413**

(87) International publication number:
**WO 2022/075460 (14.04.2022 Gazette 2022/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.10.2020 JP 2020171532**

(71) Applicants:
- **NapaJen Pharma, Inc.**
  **Seattle, Washington 98101 (US)**
- **The University of Kitakyushu**
  **Kitakyushu-shi, Fukuoka 802-8577 (JP)**

(72) Inventors:
- **ARIMA Kenji**
  **Koganei-shi Tokyo 184-0012 (JP)**
- **SAKURAI Kazuo**
  **Kitakyushu-shi Fukuoka 808-0135 (JP)**
- **SUMIYA Kazuki**
  **Kitakyushu-shi Fukuoka 808-0135 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) HOMOGENEOUS BETA-1,3-GLUCAN/NUCLEIC ACID COMPLEX AND USE THEREOF

(57) The present invention provides a population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising the homo-polynucleotide that binds to β-1,3-glucan via a hydrogen bond contained in the nucleic acid,
wherein 30% or more of the β-1,3-glucan/nucleic acid complexes contained in the population satisfy the following conditions:
(i) they are homogeneous in the length of the homo-polynucleotide contained in the nucleic acid comprising a homo-polynucleotide that bind to β-1,3-glucan via a hydrogen bond constituting the complex, and
(ii) the molecular number of the nucleic acid(s) comprising a homo-polynucleotide that binds to β-1,3-glucan via a hydrogen bond contained in one molecule of the complex is selected from the group consisting of 1, 2 and 3.

EP 4 227 416 A1

## Description

### TECHNICAL FIELD

The present invention relates to homogeneous β-1,3-glucan/nucleic acid complexes and use thereof. More particularly, the present invention relates to a homogeneous population of low-molecular-weight β-1,3-glucan/nucleic acid complexes having a triple-helical structure comprising two β-1,3-glucan chains and one nucleic acid chain comprising polydeoxyadenylic acid, and their use for introducing a nucleic acid into a cell, and the like.

### BACKGROUND ART

Schizophyllan (SPG) is an extracellular polysaccharide produced by the fungus *Schizophyllum commune.* Its chemical structure consists of linearly linked β-1,3-D-glucoses and one β-1,6-D-glucose side chain for every three main-chain glucoses (Fig. 1A). It was discovered in 1970 by Kikumoto et al., after the first discovery of its family, curdlan. When dissolved in a neutral solvent, β-1,3-glucan such as SPG or the like exhibits a semiflexible-rod-like triple-helical structure (tSPG, Fig. 1B), but when dissolved in an alkaline solvent (> 0.25N NaOH) or dimethyl sulfoxide (DMSO), the triple helix dissociates to form a single chain (sSPG). Regeneration and renaturation from sSPG to tSPG can be observed by changing the sSPG solution conditions to those favorable for tSPG, such as pH 5-8 or DMSO/water < 4 (weight ratio). While the local structure of the renatured SPG certainly exhibits the same triple helix as the original tSPG, many branches or crosslinks are observed throughout the overall morphology according to X-ray diffraction patterns. This contrasts with the original tSPG, which exhibits a straight rod shape with no branches. Circular objects are sometimes formed when the renaturation is carried out at a low concentration, which is presumably a result of an intramolecular end-to-end interaction, called "back-biting reaction" which can form circular conformations in polymerization.

One of the interesting properties of this β-1,3-D-glucan family is a formation of a polysaccharide/polynucleotide complex as shown in Fig. 1D and E. When a single-chain homo-polynucleotide such as poly(cytidylic acid) (poly(C)) and poly(deoxyadenylic acid) (poly(dA)) is present in the renaturation process, a new complex is formed instead of tSPG formation (Non-Patent Documents 1-4). This complex has the exact stoichiometric composition in which two main-chain glucoses bind to one nucleotide (Fig. 1E). By using the biological recognition of SPG by a receptor called Dectin-1, this complex has been used as a vehicle for delivering a therapeutic oligonucleotide including an antisense nucleic acid, CpG DNA, and siRNA to an antigen-presenting cell expressing this receptor (Non-Patent Documents 3, 5-8).

Among homo-polynucleotides, poly(dA)x has the highest binding affinity for SPG, and the complexation yield reaches nearly 100% when X exceeds about 30. Recently, it was found that the binding stability is further enhanced by replacing one oxygen with sulfur in the polynucleotide backbone, as shown in Fig. 1C (Non-Patent Document 9). This phosphorothioation results in the formation of more stable complexes, allowing more accurate molecular characterization of the complexes in a solution using scattering techniques (Non-Patent Document 10). Such accurate characterization is highly recommended by regulatory authorities including the U.S. Food and Drug Administration. This is because the particle size and its distribution are the most critical quality characteristics for assessing the safety and efficacy of nanomedicines. In this context, studying the properties of dilute solutions of a SPG-polynucleotide complex is indispensable not only for submitting an application to the authorities to initiate a clinical trial, but also for extending the horizon in the field of macromolecular science.

In a previous report, the present inventors have studied the properties of dilute solutions of SPG-dAx and found that this complex behaves as a semi-flexible rod (Non-Patent Document 10).

Non-patent document 11 shows that the complex of the 60-mer of deoxyadenine (dA60) with schizophyllan (SPG) (dA60/SPG) was analyzed by gel permeation chromatography and that the dA60/SPG is a mixture of several complexes with different molecular weights.

### CITATION LIST

### NON-PATENT DOCUMENTS

Non-Patent Document 1: Journal of the American Chemical Society 2000, 122 (18), 4520-4521.
Non-Patent Document 2: Biomacromolecules 2001, 2 (3), 641-650.
Non-Patent Document 3: J Am Chem Soc 2004, 126 (27), 8372-3.
Non-Patent Document 4: Chem Commun (Camb) 2005,(35), 4383-98.
Non-Patent Document 5: Bioconjugate chemistry 2017, 28 (2), 565-573.
Non-Patent Document 6: Journal of Controlled Release 2015, 220, 495-502.

Non-Patent Document 7: Proceedings of the National Academy of Sciences 2014, 111 (8), 3086-3091.
Non-Patent Document 8: International journal of molecular sciences 2020, 21 (2), 683-693.
Non-Patent Document 9: Bioorganic Chemistry 2010, 38 (6), 260-264.
Non-Patent Document 10: The Journal of Physical Chemistry 2012, 116 (1), 87-94.
Non-Patent Document 11: Koubunshi 2018, 67(7), 404-409

## SUMMARY OF THE INVENTION

### PROBLEMS TO BE SOLVED BY THE INVENTION

Although the conventionally reported complex of SPG with poly(dA) is a superior nucleic-acid-delivery vehicle, further improvement in their nucleic-acid-delivery efficiency is expected. In addition, since the conventional poly(dA)/SPG complex is a mixture of multiple forms of complexes, there is room for improvement in terms of chemical "assurance of equivalence" for developing it as a medicine, and there is a demand for providing more chemically homogeneous complexes.

The present invention aims to provide P-1,3-glucan/nucleic acid complexes that are chemically homogeneous and have superior nucleic-acid-delivery capability.

### MEANS TO SOLVE THE PROBLEMS

The present inventors have focused particularly on the low-molecular-weight SPG/poly(dA) complex and analyzed their properties in detail. SPG was lowered in molecular weight with ultrasonication or alkaline hydrolysis and subjected to an ultrafiltration membrane with a pore size of 10 kDa or 50 kDa to remove high-molecular-weight components. When the resulting low-molecular-weight SPG was complexed with dA60 and the resulting mixture of the complexes was separated by gel filtration chromatography for low-molecular-weight analysis, three peaks were clearly observed. These three peaks were fractionated and named q1, q2, and q3 from the low-molecular-weight side, and properties of the SPG/dA60 complexes in respective peaks such as molecular weight or the like were analyzed in detail by light scattering or other means. As a result, it was found that the molecular weight ratio of q1 :q2:q3 is 1 :2:3, and that one molecule of dA60 is contained in the q1 complex, two molecules of dA60 is contained in the q2 complex, and three molecules of dA60 is contained in the q3 complex. Furthermore, the ratio of the mole numbers of main-chain glucoses to the mole numbers of bases (dA) in each complex ([mG]/[dA]) was calculated and found to be almost identical to the stoichiometric ratio of 2 for each of q1, q2 and q3. These results indicate that poly(dA) choses a single-strand SPG of exactly the suitable size (length) without excess or deficiency to be able to form a complex with itself in the complex formation process, and the resulting complexes q1, q2, and q3 are homogeneous in terms of the number of dA60 molecules in one molecule of the complex, the size of the SPG expressed as a mole number of main-chain glucoses, and molecular weight.

Furthermore, when a nucleic acid was introduced into a cell using these homogeneous low-molecular-weight SPG/poly(dA) complexes, surprisingly, the efficiency of nucleic acid introduction was increased compared to that of the high-molecular-weight SPG/poly(dA) complexes. Based on these findings, the present inventors have further investigated and completed the present invention.

Accordingly, the present invention relates to the followings:

[1] A population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising the homo-polynucleotide that binds to β-1,3-glucan via a hydrogen bond contained in the nucleic acid,
wherein 30% or more of the β-1,3-glucan/nucleic acid complexes contained in the population satisfy the following conditions:

(i) they are homogeneous in the length of the homo-polynucleotide contained in the nucleic acid comprising a homo-polynucleotide that bind to β-1,3-glucan via a hydrogen bond constituting the complex, and
(ii-a) the molecular number of the nucleic acid(s) comprising a homo-polynucleotide that binds to β-1,3-glucan via a hydrogen bond contained in one molecule of the complex is 1, 2 or 3.

[2] The population of the β-1,3-glucan/nucleic acid complexes of [1], wherein 40% or more of the β-1,3-glucan/nucleic acid complexes contained in the population satisfy the conditions (i) and (ii-a).
[3] The population of the β-1,3-glucan/nucleic acid complexes of [1], wherein 50% or more of the β-1,3-glucan/nucleic acid complexes contained in the population satisfy the conditions (i) and (ii-a).

[4] The population of the β-1,3-glucan/nucleic acid complexes of [1], wherein 60% or more of the β-1,3-glucan/nucleic acid complexes contained in the population satisfy the conditions (i) and (ii-a).
[5] The population of the β-1,3-glucan/nucleic acid complexes of [1], wherein 70% or more of the β-1,3-glucan/nucleic acid complexes contained in the population satisfy the conditions (i) and (ii-a).
[6] The population of the β-1,3-glucan/nucleic acid complexes of [1], wherein 80% or more of the β-1,3-glucan/nucleic acid complexes contained in the population satisfy the conditions (i) and (ii-a).
[7] The population of the β-1,3-glucan/nucleic acid complexes of [1], wherein 90% or more of the β-1,3-glucan/nucleic acid complexes contained in the population satisfy the conditions (i) and (ii-a).
[8] The population of the β-1,3-glucan/nucleic acid complexes of any of [1] to [7], wherein the molecular number of the nucleic acid(s) comprising a homo-polynucleotide that binds to β-1,3-glucan via a hydrogen bond contained in one molecule of the complex as defined in (ii-a) is 1 or 2.
[9] The population of the β-1,3-glucan/nucleic acid complexes of any of [1] to [8], wherein 50% or more of the total number of the β-1,3-glucan/nucleic acid complexes satisfying the conditions (i) and (ii-a) are a P-1,3-glucan/nucleic acid complex in which the molecular number of the nucleic acid(s) comprising a homo-polynucleotide that binds to β-1,3-glucan via a hydrogen bond contained in one molecule of the complex is 1.
[10] A population of P-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein 30% or more of the β-1,3-glucan/nucleic acid complexes contained in the population satisfy the following conditions:

(i) they are homogeneous in the length of the homo-polynucleotide contained in the nucleic acid comprising a homo-polynucleotide that bind to β-1,3-glucan via a hydrogen bond constituting the complex, and
(ii-b) they are homogeneous in the molecular number of the nucleic acid (s) comprising a homo-polynucleotide that binds to β-1,3-glucan via a hydrogen bond contained in one molecule of the complex, wherein the molecular number of the nucleic acid(s) is 1, 2 or 3.

[11] The population of the β-1,3-glucan/nucleic acid complexes of [10], wherein 40% or more of the β-1,3-glucan/nucleic acid complexes contained in the population satisfy the conditions (i) and (ii-b).
[12] The population of the P-1,3-glucan/nucleic acid complexes of [10], wherein 50% or more of the β-1,3-glucan/nucleic acid complexes contained in the population satisfy the conditions (i) and (ii-b).
[13] The population of the β-1,3-glucan/nucleic acid complexes of [10], wherein 60% or more of the β-1,3-glucan/nucleic acid complexes contained in the population satisfy the conditions (i) and (ii-b).
[14] The population of the β-1,3-glucan/nucleic acid complexes of [10], wherein 70% or more of the β-1,3-glucan/nucleic acid complexes contained in the population satisfy the conditions (i) and (ii-b).
[15] The population of the P-1,3-glucan/nucleic acid complexes of [10], wherein 80% or more of the β-1,3-glucan/nucleic acid complexes contained in the population satisfy the conditions (i) and (ii-b).
[16] The population of the P-1,3-glucan/nucleic acid complexes of [10], wherein 90% or more of the β-1,3-glucan/nucleic acid complexes contained in the population satisfy the conditions (i) and (ii-b).
[17] The population of the P-1,3-glucan/nucleic acid complexes of any of [10] to [16], wherein the molecular number of the nucleic acid(s) comprising a homo-polynucleotide that binds to β-1,3-glucan via a hydrogen bond contained in one molecule of the complex as defined in (ii-b) is 1.
[18] The population of the β-1,3-glucan/nucleic acid complexes of any of [1] to [17], wherein the average ratio of the number of main-chain glucose units of the β-1,3-glucan to the number of nucleotide units constituting the homo-polynucleotide (number of main-chain glucose units/number of nucleotide units) contained in one molecule of the complex is 1.5 to 2.5.
[19] The population of the β-1,3-glucan/nucleic acid complexes of any of [1] to [18], wherein 30% or more of the β-1,3-glucan/nucleic acid complexes contained in the population further satisfy the following condition:
(iii) the molecular number of β-1,3-glucan(s) contained in one molecule of the complex is 1 or 2.
[20] The population of the β-1,3-glucan/nucleic acid complexes of [19], wherein 80% or more of the β-1,3-glucan/nucleic acid complexes contained in the population further satisfy the condition (iii).
[21] The population of the β-1,3-glucan/nucleic acid complexes of any of [1] to [20], wherein the length of the homo-polynucleotide contained in the nucleic acid comprising a homo-polynucleotide that bind to β-1,3-glucan via a hydrogen bond constituting the complex as defined in (i) is 20 to 100 bases.
[22] The population of the β-1,3-glucan/nucleic acid complexes of any of [1] to [21], wherein the homo-polynucleotide is polydeoxyadenylic acid.
[23] The population of the β-1,3-glucan/nucleic acid complexes of any of [1] to [22], wherein the β-1,3-glucan is

schizophyllan or curdlan.
[24] The population of the β-1,3-glucan/nucleic acid complexes of [23], wherein the β-1,3-glucan is schizophyllan.
[25] The population of the β-1,3-glucan/nucleic acid complexes of any of [1] to [24], wherein the nucleic acid comprising the homo-polynucleotide that bind to β-1,3-glucan via a hydrogen bond comprises an additional nucleic acid, and wherein the additional nucleic acid is linked to the end of the homo-polynucleotide.
[26] The population of the β-1,3-glucan/nucleic acid complexes of [25], wherein the additional nucleic acid is linked to the 5' end of the homo-polynucleotide.
[27] The population of the P-1,3-glucan/nucleic acid complexes of [25] or [26], wherein the additional nucleic acid has an expression suppressing activity against a target gene.
[28] The population of the P-1,3-glucan/nucleic acid complexes of any of [25] to [27], wherein the additional nucleic acid is an antisense nucleic acid, siRNA or miRNA.
[29] A production method of the population of the P-1,3-glucan/nucleic acid complexes of any of [1] to [28], which comprises

a step of mixing a nucleic acid comprising a homo-polynucleotide that binds to β-1,3-glucan via a hydrogen bond with β-1,3-glucan to prepare β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein the β-1,3-glucan provided for mixing has a weight-average molecular weight of 100,000 or less and has a peak area for β-1,3-glucan with a molecular weight of 100,000 or more accounting for 50% or less of the total when separated by gel permeation chromatography and detected with a differential refractive index detector.

[30] The production method of [29], wherein the ratio of the total number of main-chain glucose units contained in β-1,3-glucan provided for mixing to the total number of nucleotide units constituting the homo-polynucleotide contained in the nucleic acid provided for mixing comprising a homo-polynucleotide that binds to β-1,3-glucan via a hydrogen bond (the total number of main-chain glucose units/the total number of nucleotide units) is 4 or more.
[31] The production method of [30], wherein the total number of main-chain glucose units/the total number of nucleotide units is 16 or less.
[32] The production method of any of [29] to [31], which further comprises a step of subjecting the resulting β-1,3-glucan/nucleic acid complexes to gel permeation chromatography to isolate a β-1,3-glucan/nucleic acid complex in which the molecular number of the nucleic acid(s) comprising a homo-polynucleotide that binds to β-1,3-glucan via a hydrogen bond contained in one molecule of the complex is selected from the group consisting of 1, 2 and 3.
[33] A composition for introducing a nucleic acid into a cell, which comprises the population of the β-1,3-glucan/nucleic acid complexes of any of [1] to [28].
[34] The composition of [33], wherein the cell is a Dectin-1 positive cell.
[35] A method for introducing a nucleic acid into a cell, which comprises bringing the population of the β-1,3-glucan/nucleic acid complexes of any of [1] to [28] into contact with the cell.
[36] Use of the population of the β-1,3-glucan/nucleic acid complexes of any of [1] to [28], for introducing a nucleic acid into a cell.
[37] A composition for suppressing an expression of a target gene in a cell, which comprises the population of the P-1,3-glucan/nucleic acid complexes of [27] or [28].
[38] The composition of [37], wherein the cell is a Dectin-1 positive cell.
[39] A method for suppressing an expression of a target gene in a cell, which comprises bringing the population of the β-1,3-glucan/nucleic acid complexes of [27] or [28] into contact with the cell.
[40] Use of the population of the β-1,3-glucan/nucleic acid complexes of [27] or [28], for suppressing an expression of a target gene in a cell.

[1-1] A population of β-1,3-glucan/nucleic acid complexes, each of which comprises P-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein 80% or more of the β-1,3-glucan/nucleic acid complexes contained in the population satisfy the following conditions:

(i) they are homogeneous in the length of the homo-polynucleotide contained in the nucleic acid comprising the homo-polynucleotide that binds to β-1,3-glucan via a hydrogen bond constituting the complex, and
(ii) they are homogeneous in the molecular number of the nucleic acid(s) comprising a homo-polynucleotide

that binds to β-1,3-glucan via a hydrogen bond contained in one molecule of the complex, wherein the molecular number of the nucleic acid(s) is selected from the group consisting of 1, 2 and 3.

[2-1] The population of the β-1,3-glucan/nucleic acid complexes of [1-1], wherein the average ratio of the number of main-chain glucose units of β-1,3-glucan to the number of nucleotide units constituting the homo-polynucleotide (the number of main-chain glucose units/the number of nucleotide units) in one molecule of the complex is 1.5 to 2.5.
[3-1] The population of the β-1,3-glucan/nucleic acid complexes of [1-1] or [2-1], wherein 80% or more of the β-1,3-glucan/nucleic acid complexes contained in the population further satisfy the following condition:
(iii) the molecular number of β-1,3-glucan(s) contained in one molecule of the complex is 1 or 2.
[4-1] The population of the β-1,3-glucan/nucleic acid complexes of any of [1-1] to [3-1], wherein the molecular number of the nucleic acid(s) comprising a homo-polynucleotide that binds to β-1,3-glucan via a hydrogen bond contained in one molecule of the complex as defined in (ii) is 1.
[5-1] The population of the P-1,3-glucan/nucleic acid complexes of any of [1-1] to [4-1], wherein the length of the homo-polynucleotide contained in the nucleic acid comprising a homo-polynucleotide that bind to β-1,3-glucan via a hydrogen bond constituting the complex as defined in (i) is 20 to 100 bases.
[6-1] The population of the β-1,3-glucan/nucleic acid complexes of any of [1-1] to [5-1], wherein the homo-polynucleotide is polydeoxyadenylic acid.
[7-1] The population of the β-1,3-glucan/nucleic acid complexes of any of [1-1] to [6-1], wherein the β-1,3-glucan is schizophyllan or curdlan.
[8-1] The population of the β-1,3-glucan/nucleic acid complexes of [7-1], wherein the P-1,3-glucan is schizophyllan.
[9-1] The population of the β-1,3-glucan/nucleic acid complexes of any of [1-1] to [8-1], wherein the nucleic acid comprising a homo-polynucleotide that bind to P-1,3-glucan via a hydrogen bond comprises an additional nucleic acid, and the additional nucleic acid is linked to the end of the homo-polynucleotide.
[10-1] The population of the β-1,3-glucan/nucleic acid complexes of [9-1], wherein the additional nucleic acid is linked to the 5' end of the homo-polynucleotide.
[11-1] The population of the P-1,3-glucan/nucleic acid complexes of [9-1] or [10-1], wherein the additional nucleic acid has an expression suppressing activity against a target gene.
[12-1] The population of the P-1,3-glucan/nucleic acid complexes of any of [9-1] to [11-1], wherein the additional nucleic acid is an antisense nucleic acid, siRNA or miRNA.
[13-1] A production method of the population of the β-1,3-glucan/nucleic acid complexes of any of [1-1] to [12-1], which comprises

a step of mixing a nucleic acid comprising a homo-polynucleotide that binds to β-1,3-glucan via a hydrogen bond with β-1,3-glucan to prepare β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein the β-1,3-glucan provided for mixing has a weight-average molecular weight of 100,000 or less and has a peak area for β-1,3-glucan with a molecular weight of 100,000 or more accounting for 50% or less of the total when separated by gel permeation chromatography and detected with a differential refractive index detector.

[14-1] The production method of [13-1], wherein the ratio of the total number of main-chain glucose units contained in β-1,3-glucan provided for mixing to the total number of nucleotide units constituting the homo-polynucleotide contained in the nucleic acid provided for mixing comprising a homo-polynucleotide that binds to β-1,3-glucan via a hydrogen bond (the total number of main-chain glucose units/the total number of nucleotide units) is 4 or more.
[15-1] The production method of [14-1], wherein the total number of main-chain glucose units/the total number of nucleotide units is 16 or less.
[16-1] The production method of any of [13-1] to [15-1], which further comprises a step of subjecting the resulting β-1,3-glucan/nucleic acid complexes to gel permeation chromatography to isolate a β-1,3-glucan/nucleic acid complex in which the molecular number of the nucleic acid(s) comprising a homo-polynucleotide that binds to β-1,3-glucan via a hydrogen bond contained in one molecule of the complex is selected from the group consisting of 1, 2 and 3.
[17-1] A composition for introducing a nucleic acid into a cell, which comprises the population of the β-1,3-glucan/nucleic acid complexes of any of [1-1] to [12-1].
[18-1] The composition of [17-1], wherein the cell is a Dectin-1 positive cell.
[19-1] A method for introducing a nucleic acid into a cell, which comprises bringing the population of the β-1,3-glucan/nucleic acid complexes of any of [1-1] to [12-1] into contact with the cell.
[20-1] Use of the population of the β-1,3-glucan/nucleic acid complexes of any of [1-1] to [12-1], for introducing a

nucleic acid into a cell.

[21-1] A composition for suppressing an expression of a target gene in a cell, which comprises the population of the β-1,3-glucan/nucleic acid complexes of [11-1] or [12-1].

[22-1] The composition of [21-1], wherein the cell is a Dectin-1 positive cell.

[23-1] A method for suppressing an expression of a target gene in a cell, which comprises bringing the population of the P-1,3-glucan/nucleic acid complexes of [11-1] or [12-1] into contact with the cell.

[24-1] Use of the population of the P-1,3-glucan/nucleic acid complexes of [11-1] or [12-1], for suppressing an expression of a target gene in a cell.

EFFECT OF THE INVENTION

A chemically homogeneous population of P-1,3-glucan/nucleic acid complexes is provided by the present invention. The population of P-1,3-glucan/nucleic acid complexes of the present invention is homogeneous in terms of the number of nucleic acid molecules contained in one molecule of the complex, the size of the β-1,3-glucan expressed as the mole number of main-chain glucoses, the size of the complex or the like, and is advantageous for development as a medicine in view of chemical "assurance of equivalence". The population of β-1,3-glucan/nucleic acid complexes of the present invention also has a superior capability for delivering a nucleic acid and is useful as a carrier for introducing a nucleic acid into a cell. The present invention also provides a population of P-1,3-glucan/nucleic acid complexes which is abundant in low-molecular-weight β-1,3-glucan/nucleic acid complexes that are of superior capability for delivering a nucleic acid.

BRIEF DESCRIPTION OF THE DRAWINGS

[Fig. 1] Fig. 1 shows the chemical structure of schizophyllan (SPG). (A) Repeating unit of SPG. (B) Triple-helical structure of SPG. (C) Chemical structure of phosphorothioated poly(deoxyadenylic acid) (dAx). (D) Schematic drawing of a complex prepared from SPG and dAx. (E) Stoichiometric structure constituted from two main-chain glucoses and one dAx.

[Fig. 2] Fig. 2 shows a schematic representation of a typical structure of a complex constituting the population of complexes of the present invention.

[Fig. 3] Fig. 3 shows an example of separation of low-molecular-weight SPG or a complex of dA60 and low-molecular-weight SPG(S-3) by gel permeation chromatography(GPC). A: RI chromatogram of S-3 before and after fractionation. B: UV chromatogram of the complex before and after fractionation, and UV chromatogram of naked dA60. C: UV chromatogram after fractionation for isolating three peaks. For ease of comparison, the baseline was shifted upwards.

[Fig. 4]Fig. 4 shows a schematic drawing of the DNA template model, in which DNA acts as a template and dAx forms a complex with a SPG chain with a matched length.

[Fig. 5] Fig. 5 shows examples of a separation of complexes of dA60 and low-molecular-weight SPG (S-3) obtained with various ([mG]/[dA])mixes by GPC. A: RI chromatogram. B: UV chromatogram. C: ([mG]/[dA])f.

[Fig. 6] Fig. 6 shows examples of the separation of low-molecular-weight SPG and complexes of dA40 and low-molecular-weight SPG by GPC. A: Separation of low-molecular-weight SPG by GPC. B: Separation of a complex of dA40 and low-molecular-weight SPG by GPC.

[Fig. 7] Fig. 7 shows RI chromatograms of low-molecular-weight complexes (q1, q2, q3) obtained from SPG-S3 and YB-1AS before and after GPC fractionation.

[Fig. 8] Fig. 8 shows an example of the effect of isolated low-molecular-weight complexes (q1, q2, q3) obtained from SPG-S3 and YB-1AS on PC-9 cell viability compared with polydisperse complexes or pre-fractionated low-molecular-weight complexes.

[Fig. 9] Fig. 9 shows an example of the effect of isolated low-molecular-weight complexes (q1, q2, q3) obtained from SPG-S3 and miR-145 on PC-9 cell viability compared with polydisperse complexes or pre-fractionated low-molecular-weight complexes.

[Fig. 10] Fig. 10 shows an example of the effect of isolated low-molecular-weight complexes (q1, q2, q3) obtained from SPG-S3 and miR-143 on PC-9 cell viability compared with polydisperse complexes or pre-fractionated low-molecular-weight complexes.

[Fig. 11] Fig. 11 shows the effect of low-molecular-weight complexes of SPG-S3 and miR-145 (q1, q2, q3) and those of SPG-S3 and miR-143 (q1, q2, q3) on the viability of CCF-STTG1 cells.

[Fig. 12] Fig. 12 shows the results of Western blotting confirming the reduction of target protein expression by the low-molecular-weight complexes.

[Fig. 13] Fig. 13 shows an example the effect of an isolated low-molecular-weight complex (q1-K-ras-AS) obtained

from SPG with lowered molecular weight and K-ras-AS on PC-9 cell viability compared with polydisperse complexes (L-SPG/K-ras-AS). *$p < 0.05$, **$p < 0.01$.

[Fig. 14] Fig. 14 shows the results of Western blotting comparing the effect of a low-molecular-weight complex (q1-K-ras-AS) with polydisperse complexes (L-SPG/K-ras-AS) on K-ras protein expression in PC-9 cells.

[Fig. 15] Fig. 15 shows an example of the effect of isolated low-molecular-weight complexes (q1-K3, q2-K3, q3-K3) obtained from SPG with lowered molecular weight and K3 on the induction of IL-6 production in splenocytes compared with polydisperse complexes (L-SPG/K3) or naked K3. *$p < 0.05$, **$p < 0.01$.

DESCRIPTION OF EMBODIMENTS

(1) Homogeneous population of β-1,3-glucan/nucleic acid complexes

In one embodiment, the present invention provides a population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid (the complex population A of the present invention). The complex population A of the present invention contains an abundance of low-molecular-weight β-1,3-glucan/nucleic acid complexes, and 30% or more (preferably 40% or more, 50% or more, 60% or more, 70% or more, 80% or more, 85% or more, 86% or more, 87% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the population satisfy the following conditions:

(i) they are homogeneous in the length of the homo-polynucleotide contained in the nucleic acid comprising a homo-polynucleotide that bind to β-1,3-glucan via a hydrogen bond constituting the complex, and

(ii-a) the molecular number of the nucleic acid(s) comprising a homo-polynucleotide that binds to β-1,3-glucan via a hydrogen bond contained in one molecule of the complex is 1, 2 or 3.

In another embodiment, the present invention provides a population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two P-1,3-glucan chains and one nucleic acid chain comprising a homo-polynucleotide that binds to β-1,3-glucan via a hydrogen bond contained in the nucleic acid (the complex population B of the present invention). The complex population B of the present invention is homogeneous in the chemical properties and 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the population satisfy the following conditions:

(i) They are homogeneous in the length of the homo-polynucleotide contained in the nucleic acid comprising a homo-polynucleotide that binds to P-1,3-glucan via a hydrogen bond constituting the complex.

(ii-b) They are homogeneous in the molecular number of the nucleic acid(s) comprising a homo-polynucleotide that binds to β-1,3-glucan via a hydrogen bond contained in one molecule of the complex, wherein the molecular number is selected from the group consisting of 1, 2 and 3.

As used herein, the term "homo-polynucleotide that binds to β-1,3-glucan via a hydrogen bond" may be referred to simply as the term "homo-polynucleotide".

The term "population of β-1,3-glucan/nucleic acid complexes" refers to a group of multiple β-1,3-glucan/nucleic acid complexes. The number of β-1,3-glucan/nucleic acid complexes constituting the complex population of the present invention is generally $10^3$ or more (e.g., $10^4$ or more, $10^5$ or more, $10^6$ or more, $10^7$ or more, $10^8$ or more, $10^9$ or more, $10^{10}$ or more, $10^{11}$ or more, $10^{12}$ or more). The upper limit of the number of β-1,3-glucan/nucleic acid complexes constituting the complex population of the present invention is not particularly limited, but may be, for example, $10^{30}$ or less, $10^{29}$ or less, $10^{28}$ or less, or the like.

The term "complex" means a product obtained by association of multiple molecules via a noncovalent bond, such as electrostatic bond, van der Waals bond, hydrogen bond, hydrophobic interaction or the like, or a covalent bond. In the complex constituting the complex population of the present invention, two β-1,3-glucan chains and one nucleic acid chain containing a homo-polynucleotide are associated via a hydrogen bond between the β-1,3-glucan and the homo-polynucleotide to form a triple-helical structure, whereby the β-1,3-glucan and the nucleic acid containing the

homo-polynucleotide which binds to β-1,3-glucan via a hydrogen bond are complexed.

β-1,3-glucan refers to a polysaccharide with the structure in which D-glucoses are linearly linked by β-1,3-glycosidic bonds. The β-1,3-glucan contained in the β-1,3-glucan/nucleic acid complex constituting the complex population of the present invention is not particularly limited as long as it forms a complex with polydeoxyadenylic acid having a triple-helical structure, examples include schizophyllan, lenthinan, scleroglucan, curdlan, pachyman, grifolan, laminaran or the like. β-1,3-glucan is preferably a β-1,3-glucan containing a β-1,6-glucopyranoside branch, such as schizophyllan, lentinan or scleroglucan, or a linear β-1,3-glucan containing no branches, such as curdlan, and more preferably schizophyllan or curdlan.

Schizophyllan (SPG) can be isolated from *Schizophyllum commune.* SPG consists of a *β* -(1→3)-D-glucan main-chain, and one *β*-(1→6)-D-glucosyl side chain per three glucoses. Lentinan (LNT) is a known β-1,3-1,6-glucan and can be isolated from shiitake mushroom. Curdlan is a linear β-1,3-glucan, and can be isolated from the cultured broth of micro-organisms such as *Agrobacterium.*

The nucleic acid constituting the β-1,3-glucan/nucleic acid complex constituting the complex population A or B of the present invention comprises a homo-polynucleotide that binds to β-1,3-glucan via a hydrogen bond. The homo-polynucleotide may be single stranded. The nucleic acid comprises a single-stranded homo-polynucleotide that binds to β-1,3-glucan via a hydrogen bond as a partial structure, allowing the formation of a triple-helical structure comprising two β-1,3-glucan chains and one nucleic acid chain comprising the homo-polynucleotide. A homo-polynucleotide that binds to β-1,3-glucan via a hydrogen bond includes polydeoxyadenylic acid (polydeoxyadenosine, polydeoxyriboadenylic acid) (poly(dA)), polydeoxythymidyl acid (polydeoxythymidine, polydeoxyribothymidylic acid) (poly(dT)), polyadenylic acid (polyadenosine, polyriboadenylic acid) (poly(A)) and polycytidyl acid (polycytidine, polyribocytidyl acid) (poly(C)). Preferably, the homo-polynucleotide that binds to β-1,3-glucan via a hydrogen bond is poly(dA). The length (number of bases) of the homo-polynucleotide that binds to P-1,3-glucan via a hydrogen bond is not particularly limited, as long as it has sufficient length to allow one nucleic acid chain containing the homo-polynucleotide to form a triple-helical complex with two β-1,3-glucan (e.g., schizophilan) chains. From the viewpoint of forming a stable triple-helical structure, the length (number of bases) of the homo-polynucleotide is usually 10 or more, preferably 20 or more, more preferably 30 or more. The longer the homo-polynucleotide is, the more stable the triple-helical structure is formed with β-1,3-glucan, so theoretically there is no upper limitation, but when it is too long, the length of the nucleic acid is likely to deviate during synthesis, so the length (number of bases) of the homo-polynucleotide is usually 200 or less, preferably 100 or less, more preferably 80 or less, and even more preferably 60 or less. The length (number of bases) of the homo-polynucleotide is preferably 20-100, more preferably 30-80, more preferably 30-60 (specifically, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60).

The nucleic acid containing a homo-polynucleotide that binds to P-1,3-glucan via a hydrogen bond may comprise an additional nucleic acid linked to the end of the homo-polynucleotide. The additional nucleic acid may be linked to either the 5' or 3' end of the homo-polynucleotide, or to both the 5' and 3' ends. The additional nucleic acid may be linked to only one of the 5' or 3' end of the homo-polynucleotide. Preferably, the additional nucleic acid is linked to the 5' end of the homo-polynucleotide or only to the 5' end of the homo-polynucleotide. The additional nucleic acid may be a single-stranded or double-stranded nucleic acid. The kind of nucleic acid is not particularly limited and may be DNA, RNA, DNA/RNA chimeric nucleic acid, DNA/RNA hybrids or the like. The length (number of bases) of the additional nucleic acid is not particularly limited, but from the viewpoint of chemical homogeneity of the complexes, it is usually 1000 or less, preferably 500 or less, more preferably 100 or less, and further preferably 50 or less (e.g., 40 or less, 30 or less, 29 or less, 28 or less, 27 or less, 26 or less, 25 or less).

In one embodiment, the additional nucleic acid is a nucleic acid having an expression suppressing activity against a desired target gene. The nucleic acid may include, but is not limited to, antisense nucleic acid (e.g., antisense DNA), siRNA, miRNA, shRNA or the like. Preferably, it is an antisense nucleic acid (e.g., antisense DNA), siRNA or miRNA. An antisense nucleic acid is a single-stranded nucleic acid (preferably DNA) having a sequence complementary to the mRNA or pre-mRNA of the target gene and the length (number of bases) of which is typically around 20 (e.g., 15-30). The antisense nucleic acid usually binds to an mRNA or pre-mRNA of a target gene and suppresses the translation to a protein. siRNA is a double-stranded RNA having a complementary sequence to the mRNA of a target gene, the length (number of bases) of which is typically around 21-23. siRNA is composed of a sense strand and an antisense strand, and usually the antisense strand contains a sequence perfectly complementary to the mRNA of the target gene, and the sense strand contains a partial sequence of the mRNA sequence of the target gene. The siRNA suppresses an expression of the target gene by cleaving the target mRNA. miRNA is a single- or double-stranded RNA having a sequence partially complementary to the mRNA of the target gene, the length (number of bases) of which is typically 19-27. "Partially complementary sequence" means that the sequence is not completely complementary, but has a mismatch in some parts. A double-stranded miRNA consists of a sense and an antisense strand, in which the antisense strand usually contains a sequence partially complementary to the target gene mRNA and the sense strand contains a sequence complementary to the antisense strand. The miRNA binds to the target mRNA and suppresses its translation, thereby suppressing an expression of the target gene. An overhang may be provided at the 3' end of the sense and/or

antisense strand of the siRNA and miRNA. The overhang is usually a two-base DNA or RNA, and may be a sequence such as TT, UU or the like. The overhang is preferably two consecutive deoxythymine d(TT). When a double-stranded siRNA or miRNA is linked to the end of a homo-polynucleotide, the sense strand may be linked to the end of the homo-polynucleotide or the anti-sense strand may be linked to the end of the homo-polynucleotide. Preferably, the sense strand is linked to the end of the homo-polynucleotide. More preferably, the 3' end of the sense strand is linked to the 5' end of the homo-polynucleotide. When linking the homo-polynucleotide to the 3' end of either double-stranded siRNA or miRNA, there may be no overhang at the 3' end of the strand, or an overhang may be provided only at the 3' end of the strand to which the homo-polynucleotide is not linked. For example, when the 3' end of the sense strand is linked to the 5' end of the homo-polynucleotide, there may be no overhang at the 3' end of the sense strand and an overhang (e.g., d(TT)) may be provided only at the 3' end of the antisense strand.

The nucleic acid having an expression suppressing activity against the target gene and the homo-polynucleotide may be linked via a direct covalent bond or via a spacer sequence. A spacer sequence means a nucleotide sequence containing one or more nucleotides inserted between two closely adjacent components. The length of the spacer sequence is not particularly limited, but is usually 1-10 nucleotides in length, preferably 1-5 nucleotides in length, more preferably 1-3 nucleotides in length. In one embodiment, the nucleic acid having an expression suppressing activity against the target gene and the homo-polynucleotide are linked together without a spacer sequence (i.e., by a direct covalent bond).

The nucleic acid containing a homo-polynucleotide that binds to β-1,3-glucan via a hydrogen bond may be suitably modified to confer resistance to degradation by a nuclease (e.g., degradation by an exo- or endo-nuclease). For example, all or part of the hydroxyl groups at the 2' position of the ribonucleotides may be replaced by fluorine or methoxy group. In addition, part or all of the phosphodiester bonds in the nucleotide may be replaced by a phosphorothioate or phosphorodithioate bond. Preferably, at least a part of the phosphodiester bonds in the nucleic acid containing a homo-polynucleotide that binds to β-1,3-glucan via a hydrogen bond is replaced by a phosphorothioate bond.

In one embodiment, the nucleic acid containing a homo-polynucleotide that binds to β-1,3-glucan via a hydrogen bond contains a modification by a phosphorothioate or phosphorodithioate bond in the homo-polynucleotide (e.g., poly(dA)) that binds to P-1,3-glucan via a hydrogen bond. More preferably, all of the phosphodiester bonds contained in the homo-polynucleotide (e.g., poly(dA)) are replaced by phosphorothioate or phosphorodithioate bonds, and more preferably all of the phosphodiester bonds contained in the homo-polynucleotide (e.g., poly(dA)) are replaced by phosphorothioate bonds. The replacement with phosphorothioate bonds is expected to result in stable complexation with P-1,3-glucan, in addition to resistance to nuclease degradation.

When siRNA or miRNA is used as an additional nucleic acid, all or part of the hydroxyl groups at the 2' position of the ribonucleotides contained in the antisense strand of the siRNA or miRNA are preferably replaced by a fluorine or methoxy group. Preferably, all of the hydroxyl groups at the 2' position of the ribonucleotides contained in the antisense strand of the siRNA or miRNA are replaced by fluorine or methoxy group. The hydroxyl groups at 2' position may be replaced by either fluorine or methoxy group but the replacements may be made, for example, in such a way that the fluorine and methoxy substituents are alternately aligned.

30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population A or B of the present invention is homogeneous(identical) with respect to the length (number of bases) of the homo-polynucleotide(e.g., poly(dA)) that binds to β-1,3-glucan via a hydrogen bond contained in the nucleic acid comprising a homo-polynucleotide that bind to β-1,3-glucan via a hydrogen bond constituting the complex. That is, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the members of the complex population of the present invention contain a nucleic acid comprising "a homo-polynucleotide that binds to β-1,3-glucan via a hydrogen bond" of the same and uniform length (number of bases). The length (number of bases) of the homo-polynucleotide is usually 200 or less, preferably 100 or less, more preferably 80 or less, and further preferably 60 or less. The length (number of bases) of the homo-polynucleotide is preferably 20 to 100, more preferably 30 to 80, more preferably 30 to 60 (specifically, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60). For example, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the complex population A or B of the present invention contain a nucleic acid comprising a homo-polynucleotide (e.g., poly(dA)) with a length (number of bases) of 60. Such population of β-1,3-glucan/nucleic acid complexes that are homogeneous with respect to the length of the homo-polynucleotide can be obtained by preparing β-1,3-glucan/nucleic acid complexes using a population of

nucleic acids comprising a homo-polynucleotide that binds to β-1,3-glucan via a hydrogen bond that are homogeneous in the length of the homo-polynucleotide. The population of nucleic acids comprising a homo-polynucleotide that binds to β-1,3-glucan via a hydrogen bond that are homogeneous with respect to the length of the homo-polynucleotide can be obtained by well-known nucleic acid synthesis and column purification techniques.

For 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population A of the present invention, the molecular number of the nucleic acid(s) comprising a homo-polynucleotide that binds to β-1,3-glucan via a hydrogen bond contained in one molecule of the complex is 1, 2 or 3. That is, the total number of the P-1,3-glucan/nucleic acid complexes satisfying the condition (i) above and any of the following conditions (ii-a-1) to (ii-a-3) accounts for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population A of the present invention:

(ii-a-1) The molecular number of the nucleic acid(s) comprising a homo-polynucleotide that binds to β-1,3-glucan via a hydrogen bond contained in one molecule of the complex is 1.
(ii-a-2) The molecular number of the nucleic acid(s) comprising a homo-polynucleotide that binds to β-1,3-glucan via a hydrogen bond contained in one molecule of the complex is 2.
(ii-a-3) The molecular number of the nucleic acid(s) comprising a homo-polynucleotide that binds to β-1,3-glucan via a hydrogen bond contained in one molecule of the complex is 3.

Preferably, for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the complex population A of the present invention, the molecular number of the nucleic acid(s) comprising a homo-polynucleotide that binds to β-1,3-glucan via a hydrogen bond contained in one molecule of the complex is 1 or 2. That is, the total number of the P-1,3-glucan/nucleic acid complexes satisfying the condition (i) above and the condition (ii-a-1) or (ii-a-2) accounts for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the complex population A of the present invention.

In a preferable embodiment, 50% or more (preferably 60% or more, 70% or more, 80% or more, 90% or more, 95% or more) of the total number of the β-1,3-glucan/nucleic acid complexes satisfying the conditions (i) and (ii-a) contained in the complex population A of the present invention are the P-1,3-glucan/nucleic acid complexes in which the molecular number of the nucleic acid(s) comprising a homo-polynucleotide that binds to β-1,3-glucan via a hydrogen bond contained in one molecule of the complex is 1. Since the β-1,3-glucan/nucleic acid complex (q1) in which the molecular number of the nucleic acid(s) in one molecule of the complex is 1 has a superior capability to deliver a nucleic acid into a cell, the complex population A of the present invention containing abundant q1 is advantageous as a carrier for introducing a nucleic acid into a cell.

30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the complex population B of the present invention are homogeneous with respect to the molecular number of the nucleic acid(s) comprising a homo-polynucleotide that binds to β-1,3-glucan via a hydrogen bond contained in one molecule of the complex, wherein the molecular number is selected from the group consisting of 1, 2 and 3. That is, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population B of the present invention satisfy any condition selected from the group consisting of the following conditions in addition to the condition (i) above:

(ii-b-1) they are homogeneous with respect to the molecular number of the nucleic acid(s) comprising a homo-polynucleotide that binds to β-1,3-glucan via a hydrogen bond contained in one molecule of the complex, wherein the molecular number is 1.

(ii-b-2) they are homogeneous with respect to the molecular number of the nucleic acid(s) comprising a homo-polynucleotide that binds to β-1,3-glucan via a hydrogen bond contained in one molecule of the complex, wherein the molecular number is 2.

(ii-b-3) they are homogeneous with respect to the molecular number of the nucleic acid(s) comprising a homo-polynucleotide that binds to β-1,3-glucan via a hydrogen bond contained in one molecule of the complex, wherein the molecular number is 3.

Preferably, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population B of the present invention are homogeneous with respect to the molecular number of the nucleic acid(s) comprising a homo-polynucleotide that binds to β-1,3-glucan via a hydrogen bond contained in one molecule of the complex, wherein the molecular number is 1. That is, the β-1,3-glucan/nucleic acid complexes, each of which comprises only one molecule of the nucleic acid comprising a homo-polynucleotide that binds to β-1,3-glucan via a hydrogen bond in one molecule of the complex, account for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the complex population B of the present invention.

It is possible to determine that 30% or more (preferably 80% or more) members of a population of β-1,3-glucan/nucleic acid complexes are homogeneous with respect to the molecular number of nucleic acid(s) comprising a homo-polynucleotide that binds to β-1,3-glucan via a hydrogen bond contained in one molecule of the complex by separating the population of β-1,3-glucan/nucleic acid complexes by gel permeation chromatography and detecting them with a spectrophotometer. When the peak area for a complex containing a specific molecular number (1, 2 or 3) of nucleic acid(s) accounts for 30% or more (preferably 80% or more) of the total area, it can be assumed that 30% or more (preferably 80% or more) members of the population of the β-1,3-glucan/nucleic acid complexes are homogeneous with respect to the molecular number of the nucleic acid(s) contained in one molecule of the complex. With the spectrophotometer, the separated complexes are detected at a wavelength at which the nucleic acids contained in the complexes can be detected (e.g., 260 nm). In the present description, the percentage (%) of β-1,3-glucan/nucleic acid complexes with a specific character contained in a population of β-1,3-glucan/nucleic acid complexes is calculated based on this peak area, unless otherwise stated.

It is also possible to confirm that the molecular number of nucleic acid(s) comprising a homo-polynucleotide that binds to β-1,3-glucan via a hydrogen bond contained in one molecule of the complex is 1, 2 or 3 in 30% or more members of a population of β-1,3-glucan/nucleic acid complexes, by separating the population of the β-1,3-glucan/nucleic acid complexes by gel permeation chromatography and detecting them with a spectrophotometry. When the sum of the peak areas for the complex containing one molecule of the nucleic acid, the complex containing two molecules of the nucleic acid and the complex containing three molecules of the nucleic acid accounts for 30% or more of the total, it can be assumed that the molecular number of the nucleic acid(s) comprising a homo-polynucleotide that binds to β-1,3-glucan via a hydrogen bond contained in one molecule of the complex is 1, 2 or 3 in 30% or more members of the population of the β-1,3-glucan/nucleic acid complexes. With the spectrophotometer, the separated complexes are detected at a wavelength at which the nucleic acids contained in the complexes can be detected (e.g., 260 nm).

In one embodiment, in the complex population A or B of the present invention, the average ratio of the number of main-chain glucose units of β-1,3-glucan to the number of nucleotide units constituting the homo-polynucleotide (the number of main-chain glucose units/the number of nucleotide units) in one molecule of the complex is 1.5 to 2.5 (preferably 1.6 to 2.4, more preferably 1.7 to 2.3, further preferably 1.8 to 2.2). That is, in the complex population A or B of the present invention, the average of the ratio of the number of main-chain glucose units of β-1,3-glucan to the number of nucleotide units constituting the homo-polynucleotide (the number of main-chain glucose units/the number of nucleotide units) is approximately the stoichiometric ratio 2, and the homo-polynucleotide (e.g., poly(dA)) contained in the nucleic acid comprising a homo-polynucleotide that binds to β-1,3-glucan via a hydrogen bond is associated with a β-1,3-glucan of just the appropriate size (length) without excess or deficiency to allow complexation with itself. The average ratio of the number of main-chain glucose units/the number of nucleotide units in a population of β-1,3-glucan/nucleic acid complexes can be determined as follows. The weight-average molecular weight of the complexes in the complex population is determined by light scattering. From the molecular number of the nucleic acid(s) comprising a homo-polynucleotide that binds to β-1,3-glucan via a hydrogen bond in one molecule of the complex constituting the complex population and the molecular weight of the nucleic acid, the equivalent portion for the nucleic acid in the weight-averaged molecular weight is calculated. The β-1,3-glucan equivalent in the weight-average molecular weight is calculated by subtracting the nucleic acid equivalent from the weight-average molecular weight. The β-1,3-glucan equivalent is divided by the molecular weight of the β-1,3-glucan repeating unit and the resulting value is multiplied by the number of main-chain

glucose units in the repeating unit to calculate the number of main-chain glucose units in one molecule of the complex. From the molecular number of the nucleic acid comprising a homo-polynucleotide that binds to β-1,3-glucan via a hydrogen bond contained in one molecule of the complex, and the number of bases of the homo-polynucleotide contained in the nucleic acid, the number of nucleotide units constituting the homo-polynucleotide contained in one molecule of the complex can be calculated. By dividing the number of main-chain glucose units by the number of nucleotide units, the intended value can be obtained.

A schematic drawing of a typical structure of a complex constituting the complex population A or B of the present invention is shown in Fig. 2. The solid line represents β-1,3-glucan and the wavy line represents a homo-polynucleotide (e.g., poly(dA)) that binds to a β-1,3-glucan chain via a hydrogen bond, respectively, q1 represents a complex containing one molecule of the nucleic acid comprising a homo-polynucleotide that binds to β-1,3-glucan chain via a hydrogen bond in one molecule of the complex. q2 represents a complex containing two molecules of the nucleic acid comprising a homo-polynucleotide that binds to β-1,3-glucan chain via a hydrogen bond in one molecule of the complex. q3 represents a complex containing three molecules of the nucleic acid comprising a homo-polynucleotide that binds to β-1,3-glucan chain via a hydrogen bond in one molecule of the complex. In q1, one homo-polynucleotide chain is constituted by one homo-polynucleotide. In q2, two homo-polynucleotides are aligned in tandem to constitute one homo-polynucleotide chain of twice the length (number of bases). In q3, three homo-polynucleotides are aligned in tandem to constitute one homo-polynucleotide chain of three times the length (number of bases). In form A, the molecular number of β-1,3-glucan in one molecule of the complex is 2, which forms a triple-helical structure with one homo-polynucleotide chain. The number of main-chain glucoses in one molecule of β-1,3-glucan in the form A may be 0.75 to 1.25 times (preferably 0.8 to 1.2 times, more preferably 0.85 to 1.15 times, further preferably 0.9 to 1.1 times) the length (number of bases) of the homo-polynucleotide. In form B, one molecule of β-1,3-glucan is contained in one molecule of the complex, in which the β-1,3-glucan is folded via a hairpin structure to form two chains, which form a triple-helical structure with one homo-polynucleotide chain. In form B, the number of main-chain glucoses in one molecule of β-1,3-glucan may be 1.5 to 2.5 times (preferably 1.6 to 2.4 times, more preferably 1.7 to 2.3 times, further preferably 1.8 to 2.2 times) the length (number of bases) of the homo-polynucleotide chain. Preferably, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the members constituting the complex population of the present invention are β-1,3-glucan/nucleic acid complexes in which the molecular number of β-1,3-glucan contained in one molecule of the complex is 1 or 2.

In a preferred embodiment, 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the complex population A or B of the invention further satisfy the following condition:

(iii) the molecular number of β-1,3-glucan in one molecule of the complex is 1 or 2.

That is, the β-1,3-glucan/nucleic acid complexes containing only 1 or 2 molecules of β-1,3-glucan in one molecule of the complex account for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the complex population A or B of the present invention.

The theoretical molecular weight of the complex contained in the complex population A or B of the present invention can be represented by the following formula.

$$\text{Theoretical molecular weight} = \langle A\rangle + \langle B\rangle \times 2$$

<A> the value obtained by multiplying the molecular weight of the nucleic acid comprising a homo-polynucleotide that binds to β-1,3-glucan via a hydrogen bond by the molecular number (1, 2 or 3) of said nucleic acid contained in one molecule of the complex described in (ii-b) above

<B> The molecular weight of P-1,3-glucan having the same number of main-chain glucoses as the value obtained by multiplying the length (number of bases) of the homo-polynucleotide described in (i) above by the molecular number (1, 2 or 3) of said nucleic acid contained in one molecule of the complex described in (ii-b) above.

In one embodiment, the weight-average molecular weight of the complex population B of the present invention is in the range of the followings:

<A> + <B> x 1.5 to <A> + <B> x 2.5

(Preferably <A> + <B> x 1.6 to <A> + <B> x 2.4,
more preferably <A> + <B> x 1.7 to <A> + <B> x 2.3, and
further preferably <A> + <B> x 1.8 to <A> + <B> x 2.2).

In one embodiment, the complex population B of the present invention has a dispersion index, expressed as Mw (weight-average molecular weight)/Mn (number-average molecular weight), of 0.8 to 1.2 (preferably 0.9 to 1.1, 0.95 to 1.05, 0.96 to 1.04, 0.97 to 1.03, 0.98 to 1.02, 0.99 to 1.01). The Mw/Mn of the complex population B of the present invention can be determined by separating the complex population by GPC and analyzing it with a light scattering instrument.

In a preferred embodiment, the complex population A of the present invention is a population of β-1,3-glucan/nucleic acid complexes, each of which comprises β-1,3-glucan (e.g., schizophyllan) and a nucleic acid comprising a homo-polynucleotide (e.g., poly(dA)) that binds to β-1,3-glucan via a hydrogen bond, and has a triple-helical structure consisting of two β-1,3-glucan chains and one nucleic acid chain comprising a homo-polynucleotide (e.g., poly(dA)) that binds to β-1,3-glucan via a hydrogen bond contained in the nucleic acid,
wherein 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the population satisfy the following conditions;

(i) They are homogeneous in the length of the homo-polynucleotide (e.g., poly(dA)) contained in the nucleic acid comprising a homo-polynucleotide that bind to β-1,3-glucan via a hydrogen bond constituting the complex.
(ii-a) The molecular number of the nucleic acid(s) containing a homo-polynucleotide that binds to β-1,3-glucan via a hydrogen bond contained in one molecule of the complex is 1, 2 or 3 (preferably 1 or 2).
(iii) The molecular number of β-1,3-glucan(s) contained in one molecule of the complex is 1 or 2.

wherein the molecular number of the nucleic acid comprising a homo-polynucleotide that binds to β-1,3-glucan in one molecule of the complex is 1 in 50% or more (preferably 60% or more, 70% or more, 80% or more, 90% or more, 95% or more) of the total number of the β-1,3-glucan/nucleic acid complexes satisfying the conditions (i), (ii-a) and (iii),
wherein the length (number of bases) of the homo-polynucleotide (e.g., poly(dA)) as defined in (i) may be 20 to 100 (more preferably 30 to 80, more preferably 30 to 60 (specifically, 30, 31, 32, 33, 34, 35, 36, 37, 38, 39, 40, 41, 42, 43, 44, 45 , 46, 47, 48, 49, 50, 51, 52, 53, 54, 55, 56, 57, 58, 59 or 60),
wherein part or all of the phosphodiester bonds in the homo-polynucleotide (e.g., poly(dA)) may be replaced by a phosphorothioate bond,
wherein the nucleic acid comprising a homo-polynucleotide that binds to β-1,3-glucan via a hydrogen bond contains an additional nucleic acid, wherein the additional nucleic acid is linked to the end (preferably 5' end) of the homo-polynucleotide, wherein the additional nucleic acid may be a nucleic acid having an expression suppressing activity against a target gene, such as an antisense nucleic acid, siRNA, miRNA, or the like, and
wherein the average ratio of the number of main-chain glucose units of β-1,3-glucan to the number of nucleotide units constituting the homo-polynucleotide (the number of main-chain glucose units/the number of nucleotide units) in one molecule of the complex is 1.5 to 2.5 (preferably 1.6 to 2.4, more preferably 1.7 to 2.3, further preferably 1.8 to 2.2).

In a preferred embodiment, the complex population B of the present invention is a population of β-1,3-glucan/nucleic acid complexes, each of which comprises β-1,3-glucan (e.g., schizophyllan) and a nucleic acid comprising a homo-polynucleotide (e.g., poly(dA)) that binds to β-1,3-glucan via a hydrogen bond, and has a triple-helical structure consisting of two β-1,3-glucan chains and one nucleic acid chain comprising a homo-polynucleotide (e.g., poly(dA)) that binds to β-1,3-glucan via a hydrogen bond contained in the nucleic acid,

wherein 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the population satisfy the following conditions;

(i) They are homogeneous in the length of the homo-polynucleotide (e.g., poly(dA)) contained in the nucleic acid comprising a homo-polynucleotide that binds to β-1,3-glucan via a hydrogen bond constituting the complex.
(ii-b) They are homogeneous in the molecular number of the nucleic acid(s) comprising a homo-polynucleotide that binds to β-1,3-glucan via a hydrogen bond contained in one molecule of the complex, wherein the molecular number is 1, 2 or 3 (preferably 1).

wherein the length (number of bases) of the homo-polynucleotide (e.g., poly(dA)) in (i) may be 20 to 100 (more preferably 30 to 80, more preferably 30 to 60 (specifically 30, 31, 32, 33, 34, 35, 36, 37, 38, 39, 40, 41, 42, 43, 44, 45 , 46, 47, 48, 49, 50, 51, 52, 53, 54, 55, 56, 57, 58, 59 or 60);
wherein part or all of the phosphodiester bonds in the homo-polynucleotide (e.g., poly(dA)) may be replaced by a phosphorothioate bond;
wherein the nucleic acid comprising a homo-polynucleotide that binds to β-1,3-glucan via a hydrogen bond contains an additional nucleic acid, wherein the additional nucleic acid is linked to the end (preferably 5' end) of the homo-polynucleotide, wherein the additional nucleic acid may be a nucleic acid having an expression suppressing activity against a target gene, such as an antisense nucleic acid, siRNA, miRNA, or the like;
wherein the average ratio of the number of main-chain glucose units of β-1,3-glucan to the number of nucleotide units constituting the homo-polynucleotide (the number of main-chain glucose units/the number of nucleotide units) contained in one molecule of the complex is 1.5 to 2.5 (preferably 1.6 to 2.4, more preferably 1.7 to 2.3, further preferably 1.8 to 2.2);
wherein 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the β-1,3-glucan/nucleic acid complexes contained in the complex population may satisfy the following condition; and

(iii) the molecular number of β-1 ,3-glucan(s) contained in one molecule of the complex is 1 or 2.

The dispersion index expressed as Mw (weight-average molecular weight)/Mn (number-average molecular weight) is 0.8 to 1.2 (preferably 0.9 to 1.1, 0.95 to 1.05, 0.96 to 1.04, 0.97 to 1.03, 0.98 to 1.02, 0.99 to 1.01).

In a preferred embodiment, the complex population B of the present invention is a population of β-1,3-glucan/nucleic acid complexes, each of which comprises β-1,3-glucan (e.g., schizophyllan) and a nucleic acid comprising polydeoxyadenylic acid, and has a triple-helical structure consisting of two β-1,3-glucan chains and one nucleic acid chain comprising polydeoxyadenylic acid, wherein 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the P-1,3-glucan/nucleic acid complexes contained in the population satisfy the following conditions;

(i) They are homogeneous in the length of the polydeoxyadenylic acid contained in the nucleic acid comprising the polydeoxyadenylic acid constituting the complex.
(ii-b-1) They are homogeneous in the molecular number of the nucleic acid(s) comprising polydeoxyadenylic acid contained in one molecule of the complex, wherein the molecular number is 1.

wherein the length (number of bases) of the polydeoxyadenylic acid is 20 to 100 (more preferably 30 to 80, more preferably 30 to 60 (specifically, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60);
wherein part or all of the phosphodiester bonds in the polydeoxyadenylic acid is replaced by phosphorothioate bonds;
wherein the nucleic acid comprising polydeoxyadenylic acid contains an additional nucleic acid, wherein the additional nucleic acid is linked to the end (preferably 5' end) of the polydeoxyadenylic acid, wherein the additional nucleic acid may be a nucleic acid having an expression suppressing activity against a target gene, such as an antisense nucleic acid, siRNA, miRNA, or the like;
wherein the average ratio of the number of main-chain glucose units of β-1,3-glucan to the number of nucleotide units constituting the polydeoxyadenylic acid (the number of main-chain glucose units/the number of nucleotide units) contained in one molecule of the complex is 1.5 to 2.5 (preferably 1.6 to 2.4, more preferably 1.7 to 2.3, further preferably 1.8 to 2.2);
wherein 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or

more, 99.5% or more, 99.9% or more) of the P-1,3-glucan/nucleic acid complexes contained in the complex population may further satisfy the following condition; and (iii) the molecular number of β-1,3-glucan(s) contained in one molecule of the complex is 1 or 2.

The dispersion index expressed as Mw (weight-average molecular weight)/Mn (number-average molecular weight) is 0.8 to 1.2 (preferably 0.9 to 1.1, 0.95 to 1.05, 0.96 to 1.04, 0.97 to 1.03, 0.98 to 1.02, 0.99 to 1.01).

(2) Production of homogeneous population of β-1,3-glucan/nucleic acid complexes

The present invention provides a production method of the population A or B of the P-1,3-glucan/nucleic acid complexes of the present invention described above (the production method of the present invention). The production method of the present invention comprises the following step:
a step of mixing a nucleic acid comprising a homo-polynucleotide that binds to β-1,3-glucan via a hydrogen bond with β-1,3-glucan to prepare β-1,3-glucan/nucleic acid complexes, each of which comprises β-1,3-glucan and the nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to P-1,3-glucan via a hydrogen bond.

The (population of) nucleic acids comprising a homo-polynucleotide that binds to β-1,3-glucan via a hydrogen bond provided for mixing are homogeneous with respect to the length (number of bases) of the homo-polynucleotide (e.g., poly(dA)) that binds to β-1,3-glucan via a hydrogen bond, and 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more, 93% or more, 94% or more, 95% or more, 96% or more, 97% or more, 98% or more, 99% or more, 99.5% or more, 99.9% or more) of the members of the population comprise "a homo-polynucleotide that binds to β-1,3-glucan via a hydrogen bond" of the same and uniform length (number of bases).

Preferably, the β-1,3-glucan provided for mixing has been subjected to a treatment for lowering the molecular weight. Ultrasonication, alkaline hydrolysis, acid hydrolysis, enzymatic treatment, or the like may be exemplified as the treatment for lowering the molecular weight. β-1,3-glucan can be efficiently lowered in molecular weight by ultrasonication under alkaline conditions, where β-1,3-glucan dissociates from triple-strand to single-strand. During the ultrasonication process, the β-1,3-glucan may be heated to around 40-50°C. For example, β-1,3-glucan is subjected to a treatment for lowering the molecular weight until the weight-average molecular weight of β-1,3-glucan becomes 100,000 or less (preferably 50,000 or less, more preferably 30,000 or less). In a preferred embodiment, β-1,3-glucan (a population of β-1,3-glucans) with a weight-average molecular weight of 100,000 or less (preferably 50,000 or less, more preferably 30,000 or less) is provided for mixing. The weight-average molecular weight of the β-1,3-glucan (the population of β-1,3-glucans) to be provided for mixing may generally be 3000 or more (preferably 4000 or more, 5000 or more, 6000 or more, 7000 or more, 8000 or more, 9000 or more, 10,000 or more).

Since a high-molecular-weight β-1,3-glucan prevents homogeneous β-1,3-glucan/nucleic acid complexes by complexing with large amounts of nucleic acid molecules to remove the nucleic acid molecules from the reaction system, β-1,3-glucan may be subjected to a treatment to remove high-molecular-weight β-1,3-glucan in addition to the treatment for lowering the molecular weight. As such treatments, ultrafiltration, gel permeation chromatography, dialysis, or the like may be included. For example, at least a fraction with a weight-average molecular weight of 100,000 or more (preferably 50,000 or more) may be removed. For example, high-molecular-weight β-1,3-glucan is removed so that the peak area for β-1,3-glucan with a molecular weight of 100,000 or more (preferably 50,000 or more) accounts for 50% or less (preferably 40% or less, 30% or less, 20% or less, 10% or less, 10% or less, and 5% or less) of the total, when the β-1,3-glucan is separated by gel permeation chromatography and detected with a differential refractive index detector. In a preferred embodiment, β-1,3-glucan (a population of β-1,3-glucans) provided for mixing has a peak area for β-1 ,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector. In a more preferred embodiment, β-1,3-glucan (a population of β-1,3-glucans) provided for mixing has a weight-average molecular weight of 100,000 or less (preferably 50,000 or less, more preferably 30,000 or less) and has a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector.

Since β-1,3-glucan exists as a triple-helical structure in nature, the low-molecular-weight β-1,3-glucan obtained as described above is dissolved in a non-protonic organic polar solvent (dimethyl sulphoxide (DMSO), acetonitrile, acetone, or the like) or an aqueous alkaline solution (sodium hydroxide, hydroxide potassium, ammonia, calcium hydroxide, or the like) to be dissociated into a single strand. The solution of the single-stranded β-1,3-glucan thus obtained is mixed with a solution (an aqueous solution, a buffered aqueous solution with a pH around neutral, or an acidic buffered

aqueous solution, preferably an aqueous solution or a buffered aqueous solution with a pH around neutral) of the above-described nucleic acid comprising a homo-polynucleotide (e.g., poly(dA)) that binds to β-1,3-glucan via a hydrogen bond, and kept for a suitable time, for example overnight at 4°C, optionally after adjusting the pH again to near neutral. As a result, a β-1,3-glucan/nucleic acid complex having a triple-helical structure consisting of two β-1,3-glucan chains and one nucleic acid chain comprising a homo-polynucleotide that binds to β-1,3-glucan via a hydrogen bond is formed.

When the complex population A of the present invention is produced, the resulting β-1,3-glucan/nucleic acid complexes may be subjected to a gel permeation chromatography to isolate a β-1,3-glucan/nucleic acid complex fraction (q1+q2+q3, preferably q1+q2) in which the molecular number of the nucleic acid(s) comprising a homo-polynucleotide that binds to β-1,3-glucan via a hydrogen bond contained in one molecule of the complex is 1 to 3 (preferably 1 to 2). This purification process removes unreacted β-1,3-glucan and nucleic acids; and complexes in which the molecular number of the nucleic acid(s) comprising a homo-polynucleotide that binds to β-1,3-glucan via a hydrogen bond in one molecule of the complex exceeds 4, thereby intended complex population A of the present invention can be obtained. For example, a fraction of the complexes in which the molecular number of the contained nucleic acid(s) is 1 to 3 (preferably 1 to 2) is isolated so that the sum of the peak areas for the complex in which the molecular number of the contained nucleic acid(s) is 1, the complex in which the molecular number of the contained nucleic acid(s) is 2, and the complex in which the molecular number of the contained nucleic acid(s) is 3 (preferably the sum of the peak areas for the complex in which the molecular number of the contained nucleic acid(s) is 1 and the complex in which the molecular number of the contained nucleic acid(s) is 2) accounts for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more , 93% or more, 94% or more, 95% or more, 96% or more, 97% or more, 98% or more, 99% or more, 99.5% or more, 99.9% or more) of the total, when the purified population of the β-1,3-glucan/nucleic acid complexes is separated by gel permeation chromatography and detected with a spectrophotometer.

When the complex population B of the present invention is produced, the resulting β-1,3-glucan/nucleic acid complexes may be subjected to a gel permeation chromatography to isolate the β-1,3-glucan/nucleic acid complexes in which the molecular number of the nucleic acid(s) comprising a homo-polynucleotide that binds to β-1,3-glucan via a hydrogen bond contained in one molecule of the complex is selected from the group consisting of 1, 2 and 3 (preferably 1). This purification process removes unreacted β-1,3-glucan and nucleic acids; and complexes in which the molecular number of the nucleic acid(s) comprising a homo-polynucleotide that binds to β-1,3-glucan via a hydrogen bond in one molecule of the complex exceeds 4, thereby intended complex population B of the present invention can be obtained. For example, the complexes which contain a specific molecular number (1, 2 or 3, preferably 1) of nucleic acid(s) comprising a homo-polynucleotide that binds to β-1,3-glucan via a hydrogen bond are fractionated so that the sum of the peak areas for the complexes which contain a specific molecular number (1, 2 or 3, preferably 1) of nucleic acid(s) comprising a homo-polynucleotide that binds to β-1,3-glucan via a hydrogen bond account for 30% or more (preferably 40% or more, 50% or more, 60% or more, 70% or more, 80% or more, 85% or more, 86% or more, 87% or more, 88% or more, 88% or more, 89% or more, 90% or more, 91% or more, 92% or more , 93% or more, 94% or more, 95% or more, 96% or more, 97% or more, 98% or more, 99% or more, 99.5% or more, 99.9% or more) of the total, when the purified population of the β-1,3-glucan/nucleic acid complexes is separated by gel permeation chromatography and detected with a spectrophotometer.

This purification process is not essential, and the production method of the present invention may not comprise this purification process. Particularly, when β-1,3-glucan (a population of β-1,3-glucans) having a weight-average molecular weight of 100,000 or less (preferably 50,000 or less, more preferably 30,000 or less); β-1,3-glucan (a population of β-1,3-glucans) having a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector; or β-1,3-glucan (a populations of β-1,3-glucans) having a weight-average molecular weight of 100,000 or less (preferably 50,000 or less, more preferably 30,000 or less) and having a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector is used as a starting material, β-1,3-glucan/nucleic acid complexes in which the number of nucleic acid molecule(s) in one molecule of the complex is selected from the group consisting of 1, 2 and 3 (preferably 1) are formed at a high yield. Accordingly, the complex population A or B of the present invention can be obtained even without the above purification process.

Although β-1,3-glucan with lowered molecular weight is usually a mixture of β-1,3-glucans with various lengths, a homo-polynucleotide (e.g., poly(dA)) that binds to β-1,3-glucan via a hydrogen bond forms a complex preferentially with the most suitable β-1,3-glucan in terms of chain length in the process of complex formation with a nucleic acid comprising the homo-polynucleotide (the homo-polynucleotide (e.g., poly(dA))-templated complex formation). As a result, a population of β-1,3-glucan/nucleic acid complexes purified on the basis of the molecular number of the nucleic acid(s) comprising a homo-polynucleotide that binds to β-1,3-glucan via a hydrogen bond contained in one molecule of the

complex may give an approximately stoichiometric value (i.e., 2), for example 1.5 to 2.5 (preferably 1.6 to 2.4, more preferably 1.7 to 2.3, even more preferably 1.8 to 2.2) as the average ratio of the number of main-chain glucose units of β-1,3-glucan to the number of nucleotide units constituting the homo-polynucleotide (the number of main-chain glucose units/the number of nucleotide units) in one molecule of the complex.

In one embodiment, β-1,3-glucans (a population of β-1,3-glucans) provided for mixing contain a β-1,3-glucan whose main-chain glucose number is 0.75 to 1.25 times (preferably 0.8 to 1.2 times, more preferably 0.85 to 1.15 times, further preferably 0.9 to 1.1 times) of the length (number of bases) of the homo-polynucleotide contained in the nucleic acid, provided for mixing, comprising a homo-polynucleotide that binds to β-1,3-glucan via a hydrogen bond and/or a β-1,3-glucan whose main-chain glucose number is 1.5 to 2.5 times (preferably 1.6 to 2.4 times, more preferably 1.7 to 2.3 times, further preferably 1.8 to 2.2) of the length (number of bases) of said homo-polynucleotide. By mixing these β-1,3-glucans (a population of β-1,3-glucans) with the nucleic acids comprising a homo-polynucleotide that binds to β-1,3-glucan via a hydrogen bond, complexes of the forms shown as q1 A and B in Fig. 2 are preferentially formed. By isolating and purifying these complexes, it is possible to obtain a population of β-1,3-glucan/nucleic acid complexes in which the average ratio of the number of main-chain glucose units of β-1,3-glucan to the number of nucleotide units constituting the homo-polynucleotide (the number of main-chain glucose units/the number of nucleotide units) in one molecule of the complex is 1.5 to 2.5 (preferably 1.6 to 2.4 times, more preferably 1.7 to 2.3 times, further preferably 1.8 to 2.2 times).

In one embodiment, the ratio of the total number of main-chain glucose units contained in β-1,3-glucan provided for mixing to the total number of nucleotide units constituting the homo-polynucleotide contained in the nucleic acid comprising a homo-polynucleotide that binds to β-1,3-glucan via a hydrogen bond provided for mixing (the total number of main-chain glucose units/the total number of nucleotide units) is set to 4 or more. By setting the total number of main-chain glucose units/the total number of nucleotide units to 4 or more, the amount of unreacted nucleic acids remained in the reaction product can be reduced.

From the viewpoint of reducing the amount of unreacted β-1,3-glucan remained in the reaction product, the ratio of the total number of main-chain glucose units contained in the β-1,3-glucan provided for mixing to the total number of nucleotide units constituting the homo-polynucleotide contained in the nucleic acid provided for mixing (the total number of main-chain glucose units/the total number of nucleotide units) is preferably 16 or less (more preferably 8 or less). That is, the total number of main-chain glucose units/the total number of nucleotide units is preferably 4 to 16, more preferably 4 to 8.

Formation of the complex can be confirmed by measuring, for example, conformational changes by CD (circular dichroism) spectroscopy, UV absorption shift by size-exclusion chromatography, gel electrophoresis, microchip electrophoresis, capillary electrophoresis, though the method is not limited thereto. Unreacted β-1,3-glucan or a nucleic acid comprising a homo-polynucleotide that binds to β-1,3-glucan via a hydrogen bond may be removed using anion chromatography or other methods.

For the production of complexes, JP2008-100919A; WO2015/04131; Japanese Patent No. 5605793; Sakurai et al, Biomacromolecules 2, 641-650 (2001); Shimada et al, Bioconjugate chemistry 18, 1280-1286 (2007); Koyama et al, Science translational medicine 2, 25ra24 (2010); Minari et al, Bioconjugate chemistry 22, 9-15 (2011), or the like can be also referred to.

In a preferred embodiment, the production method of the present invention comprises the following steps:

a step of mixing a nucleic acid comprising a homo-polynucleotide (e.g., poly(dA)) that binds to P-1,3-glucan via a hydrogen bond with β-1,3-glucan (e.g., schizophyllan) to prepare β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan (e.g., schizophyllan) chains and one nucleic acid chain comprising a homo-polynucleotide (e.g., poly(dA)) that binds to β-1,3-glucan via a hydrogen bond contained in the nucleic acid, and

a step of subjecting the resulting β-1,3-glucan/nucleic acid complexes to gel permeation chromatography to isolate a β-1,3-glucan/nucleic acid complex fraction (q1+q2+q3, preferably q1+q2) in which the molecular number of the nucleic acid(s) comprising a homo-polynucleotide that binds to β-1,3-glucan via a hydrogen bond contained in one molecule of the complex is 1 to 3 (preferably 1 to 2);

wherein the β-1,3-glucan provided for mixing is β-1,3-glucan (a populations of β-1,3-glucans) having a weight-average molecular weight of 100,000 or less (preferably 50,000 or less, more preferably 30,000 or less); β-1,3-glucan (a population of β-1,3-glucans) having a peak area for a β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector; or β-1,3-glucan (a populations of β-1,3-glucans) having a weight-average molecular weight of 100,000 or less (preferably 50,000 or less, more preferably 30,000 or less) and having a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel

permeation chromatography and detected with a differential refractive index detector;
wherein the β-1,3-glucan (a population of β-1,3-glucans) provided for mixing may contain β-1,3-glucan whose main-chain glucose number is 0.75 to 1.25 times (preferably 0.8 to 1.2 times, more preferably 0.85 to 1.15 times, further preferably 0.9 to 1.1 times) of the length (number of bases) of the homo-polynucleotide and/or β-1,3-glucan whose main-chain glucose number is 1.5 to 2.5 times (preferably 1.6 to 2.4 times, more preferably 1.7 to 2.3 times, further preferably 1.8 to 2.2) of the length (number of bases) of the homo-polynucleotide; and
wherein the ratio of the total number of main-chain glucose units contained in the β-1,3-glucan provided for mixing to the total number of nucleotide units constituting the homo-polynucleotide contained in the nucleic acid provided for mixing comprising a homo-polynucleotide that binds to β-1,3-glucan via a hydrogen bond (the total number of main-chain glucose units/the total number of nucleotide units) may be 4 to 16 (preferably 4 to 8).

In a preferred embodiment, the production method of the present invention comprises the following steps:

a step of mixing a nucleic acid comprising a homo-polynucleotide (e.g., poly(dA)) that binds to β-1 ,3-glucan via a hydrogen bond with β-1,3-glucan (e.g., schizophyllan) to prepare β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan (e.g., schizophyllan) chains and one nucleic acid chain comprising a homo-polynucleotide (e.g., poly(dA)) that binds to β-1,3-glucan via a hydrogen bond contained in the nucleic acid, and
a step of subjecting the resulting β-1,3-glucan/nucleic acid complexes to gel permeation chromatography to isolate a β-1,3-glucan/nucleic acid complex in which the molecular number of the nucleic acid(s) comprising a homo-polynucleotide that binds to β-1 ,3-glucan via a hydrogen bond contained in one molecule of the complex is selected from the group consisting of 1, 2 and 3 (preferably 1);
wherein the β-1,3-glucan provided for mixing is β-1,3-glucan (a populations of β-1,3-glucans) having a weight-average molecular weight of 100,000 or less (preferably 50,000 or less, more preferably 30,000 or less); β-1,3-glucan (a population of β-1,3-glucans) having a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector; or β-1,3-glucan (a populations of β-1,3-glucans) having a weight-average molecular weight of 100,000 or less (preferably 50,000 or less, more preferably 30,000 or less) and having a peak area for β-1,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector;
wherein the β-1,3-glucan (a population of β-1,3-glucans) provided for mixing may contain β-1,3-glucan whose main-chain glucose number is 0.75 to 1.25 times (preferably 0.8 to 1.2 times, more preferably 0.85 to 1.15 times, further preferably 0.9 to 1.1 times) of the length (number of bases) of the homo-polynucleotide and/or β-1,3-glucan whose main-chain glucose number is 1.5 to 2.5 times (preferably 1.6 to 2.4 times, more preferably 1.7 to 2.3 times, further preferably 1.8 to 2.2) of the length (number of bases) of the homo-polynucleotide; and
wherein the ratio of the total number of main-chain glucose units contained in the β-1,3-glucan provided for mixing to the total number of nucleotide units constituting the homo-polynucleotide contained in the nucleic acid provided for mixing comprising a homo-polynucleotide that binds to β-1,3-glucan via a hydrogen bond (the total number of main-chain glucose units/the total number of nucleotide units) may be 4 to 16 (preferably 4 to 8).

In a preferred embodiment, the production method of the present invention comprises the following steps:

a step of mixing a nucleic acid comprising polydeoxyadenylic acid with β-1,3-glucan (e.g., schizophyllan) to prepare β-1 ,3-glucan/nucleic acid complexes, each of which comprises β-1,3-glucan and a nucleic acid comprising polydeoxyadenylic acid, and has a triple-helical structure consisting of two β-1,3-glucan (e.g., schizophyllan) chains and one nucleic acid chain comprising polydeoxyadenylic acid, and
a step of subjecting the resulting β-1,3-glucan/nucleic acid complexes to gel permeation chromatography to isolate β-1,3-glucan/nucleic acid complex in which the molecular number of the nucleic acid(s) comprising polydeoxyadenylic acid contained in one molecule of the complex is 1;
wherein the β-1,3-glucan provided for mixing has a weight-average molecular weight of 100,000 or less (preferably 50,000 or less, more preferably 30,000 or less) and has a peak area for β-1 ,3-glucan with a molecular weight of 100,000 or more (preferably 50,000 or more) accounting for 50% or less (preferably 40% or less, 30% or less, 20% or less, 10% or less, 5% or less) of the total when separated by gel permeation chromatography and detected with a differential refractive index detector;
wherein the β-1,3-glucan (a population of β-1,3-glucans) provided for mixing contains a β-1,3-glucan whose main-chain glucose number is 0.75 to 1.25 times (preferably 0.8 to 1.2 times, more preferably 0.85 to 1.15 times, further preferably 0.9 to 1.1 times) of the length (number of bases) of the polydeoxyadenylic acid and/or a β-1,3-glucan whose main-chain glucose number is 1.5 to 2.5 times (preferably 1.6 to 2.4 times, more preferably 1.7 to 2.3 times, further preferably 1.8 to 2.2) of the length (number of bases) of the polydeoxyadenylic acid; and
wherein the ratio of the total number of main-chain glucose units contained in the β-1,3-glucan provided for mixing to the total number of nucleotide units constituting the polydeoxyadenylic acid contained in the nucleic acid provided for mixing (the total number of main-chain glucose units/the total number of nucleotide units) is 4 to 16 (preferably 4 to 8).

The definition of each term in this section is as described above in "(1) homogeneous population of β-1,3-glucan/nucleic acid complexes", unless otherwise stated.

(3) Use for introducing a nucleic acid

The populations A and B of the β-1,3-glucan/nucleic acid complexes of the present invention have an excellent nucleic-acid-delivery capability and are useful as a carrier for introducing a nucleic acid into a cell. That is, the present invention provides a composition for introducing a nucleic acid into a cell, comprising the population A or B of the β-1,3-glucan/nucleic acid complexes of the present invention described above. By bringing the population of β-1,3-glucan/nucleic acid complexes of the present invention in contact with a cell in vivo or in vitro, the nucleic acid is introduced into the cell. The amount and method of introduction of the complex population A or B of the present invention into the cell is same as in the case of conventional nucleic acid introduction. Since the complex populations A and B of the present invention show superior intracellular transfer capability even when used alone, it is possible to introduce a nucleic acid into a cell without using conventional gene transfer reagents used for the introduction of a nucleic acid into a cell, or by reducing the amount of conventional gene transfer reagents used. By administering an effective amount of the population A or B of the β-1,3-glucan/nucleic acid complexes of the present invention to a subject animal (e.g., a mammal such as a human), the nucleic acid can be introduced into a cell in the subject animal. The present invention also provides a use of the population of β-1,3-glucan/nucleic acid complexes of the present invention for introducing a nucleic acid into a cell in vitro. The nucleic acid to be introduced into the cell is a nucleic acid containing a homo-polynucleotide that binds to β-1,3-glucan via a hydrogen bond contained in the β-1,3-glucan/nucleic acid complex.

Preferably, the cell is a cell expressing Dectin-1, a receptor for β-1,3-glucan, on the cell surface (Dectin-1-positive cell). β-1,3-glucan contained in the β-1,3-glucan/nucleic acid complex binds to Dectin-1 on the cell surface, thereby the β-1,3-glucan/nucleic acid complex is incorporated into the cell and the nucleic acid contained in the complex is introduced into the cell. As a Dectin-1 positive cell, macrophage, dendritic cell, neutrophil, or the like can be mentioned, but are not limited to. Preferably, the cell is a Dectin-1-positive cell of a mammal, such as a human.

An expression of a target gene can be suppressed in a cell by using a nucleic acid comprising a homo-polynucleotide that binds to β-1,3-glucan via a hydrogen bond and an additional nucleic acid, in which the additional nucleic acid is linked to the end of the homo-polynucleotide, and the additional nucleic acid has an expression suppressing activity against the target gene as the nucleic acid contained in the population A or B of the β-1,3-glucan/nucleic acid complexes of the present invention. The present invention provides a composition for suppressing an expression of a target gene in a cell, which comprises the population A or B of the β-1,3-glucan/nucleic acid complexes of the present invention described above, wherein the nucleic acid contained in the complexes comprises a homo-polynucleotide that binds to β-1,3-glucan via a hydrogen bond and an additional nucleic acid, wherein the additional nucleic acid is linked to the end of the homo-polynucleotide, and wherein the additional nucleic acid has an expression suppressing activity against the target gene. The expression of a target gene in a cell can be suppressed by bringing the population A or B of the β-1,3-glucan/nucleic acid complexes of the present invention into contact with the cell in vitro or in vivo, wherein the nucleic acid contained in the complexes comprises a homo-polynucleotide that binds to β-1,3-glucan via a hydrogen bond and an additional nucleic acid, wherein the additional nucleic acid is linked to the end of the homo-polynucleotide, and wherein the additional nucleic acid has an expression suppressing activity against the target gene. An expression of a target gene in a cell in a subject animal can be suppressed by administering an effective amount of the population A or B of the β-1,3-glucan/nucleic acid complexes of the present invention to the target animal (e.g., a mammal such as a human), wherein the nucleic acid contained in the complexes comprises a homo-polynucleotide that binds to β-1,3-glucan via a hydrogen bond and an additional nucleic acid, wherein the additional nucleic acid is linked to the end of the homo-polynucleotide, and wherein the additional nucleic acid has an expression suppressing activity against the target gene. The present invention also provides a use of the population A or B of the β-1,3-glucan/nucleic acid complexes of the present invention for suppressing an expression of a target gene in a cell in vitro, wherein the nucleic acid contained in the complexes comprises a homo-polynucleotide that binds to β-1,3-glucan via a hydrogen bond and an additional nucleic acid, wherein the additional nucleic acid is linked to the end of the homo-polynucleotide, and wherein the additional nucleic acid has an expression suppressing activity against the target gene. Preferably, the cell is a cell expressing

Dectin-1, a receptor for β-1,3-glucan, on the cell surface (Dectin-1-positive cell).

The target gene is not particularly limited, but from the viewpoint of use for pharmaceutical purpose, a gene which is involved in a pathological condition and whose expression is desired to be suppressed are suitable. Specific examples of the target gene include (a) a gene encoding a factor involved in the onset of a pathological condition or aggravation of symptoms by overproduction of its transcript through external stimulation, or the like; (b) a gene encoding a factor in which the target gene has a mutated site and whose transcript is directly involved in the onset of the disease. The target gene is preferably a gene expressed by a Dectin-1-positive cell. As the target genes mentioned above, genes encoding a factor that induces inflammation, such as cytokines including TNFα, interleukins, MIF, etc., can be exemplified. The case where the target gene in (a) is a gene encoding a factor that controls on/off of intracellular activity is also encompassed. As such genes, protein kinases (e.g., Raf, MEK, Jak2, KRAS, or the like) and transcription factors (e.g., Stat5, YB-1, or the like) can be exemplified. Furthermore, the case where the target gene in (a) is a gene encoding a cell surface receptor and a factor that acts on the onset of a pathological condition or aggravation of a disease by said cell surface receptor is also encompassed. As such genes, genes encoding TNFR (tumour necrosis factor receptor), PDGFR (platelet-derived growth factor receptor), interleukin receptors or the like can be exemplified.

The pharmaceutical composition of the present invention may contain a pharmacologically acceptable carrier in addition to the population A or B of the β-1,3-glucan/nucleic acid complexes of the present invention. Such pharmaceutical composition is provided in a dosage form suitable for oral or parenteral administration.

As a composition for parenteral administration, for example, injection, suppository or the like may be used, and the injection may include dosage forms such as intravenous injection, subcutaneous injection, intradermal injection, muscular injection, drip injection and the like. Such injections can be prepared according to a known method. The preparation method of the injection includes dissolving or suspending the complex population of the present invention in a sterile aqueous solvent generally used for injection. As the aqueous solvent for injection, for example, distilled water; physiological saline; buffers such as phosphate buffer, carbonate buffer, tris buffer, acetate buffer or the like can be used. The pH of such aqueous solvent is, for example, 5 to 10, preferably 6 to 8. Prepared injection is preferably filled in a suitable ampoule.

A powder preparation comprising the complex population of the present invention may also be prepared by subjecting a solution or a suspension comprising the complex population A or B of the present invention to vacuum drying, freezedrying, or other treatment. The complex population A or B of the present invention can be provided for use by storing same in a powder form and dissolving or dispersing the powder in an aqueous solvent for injection at the time of use.

The content of the complex population A or B of the present invention in the pharmaceutical composition is, for example, about 0.1 to 100 wt%, preferably about 1 to 99 wt%, more preferably about 10 to 90 wt% of the whole pharmaceutical composition.

The definition of each term in this section is as described above in "(1) homogeneous population of β-1,3-glucan/nucleic acid complexes" and "(2) Production of homogeneous population of β-1,3-glucan/nucleic acid complexes", unless otherwise stated.

All references cited in the present specification, including publication, patent document and the like, are hereby incorporated individually and specifically by reference, to the extent that the entireties thereof have been specifically disclosed herein.

The present invention is explained in more detail in the following by referring to Examples, which are not to be construed as limitative.

## EXAMPLES

### [Experimental Example 1] Lowering the molecular weight of β-1,3-glucan

Triple-helical schizophyllan (SPG) having a weight-average molecular weight of 1.5 million as determined by light scattering, was dissolved in 8 mL of 0.01N NaOH aqueous solution to a concentration of 10 mg/mL (maintaining the triple helix), adjusted to neutral with a phosphate buffer, and then sonicated for 2 hours using S-4000-astrasonics (frequency 20 kHz, output 600 W). The sample was then lyophilized to obtain a solid form low-molecular-weight SPG. This sample was designated SPG-S1a. SPG with a weight-average molecular weight of 800,000 was treated under a similar condition, and the resulting low-molecular-weight SPG was designated SPG-S3a.

1 mL of 2.5N NaOH aqueous solution was added to 5 mg of SPG with a weight-average molecular weight of 1.5 million, and the obtained mixture was heated and stirred at 60°C. The mixture was then neutralized with hydrochloric acid and lyophilized to obtain a solid of low-molecular-weight SPG. This sample was designated SPG-S2a. In addition, SPG with a weight-average molecular weight of 800,000 was treated under a similar condition, and the resulting low-molecular-weight SPG was designated as SPG-S4a. The similar alkali treatment was performed on cardran (4987481235458) (CUR) manufactured by Fujifilm Wako, and the resulting low-molecular-weight CUR was designated

CUR-S1a.

SPG-S1a to 4a and CUR-S1a were added to a 0.25N NaOH aqueous solution to a concentration of 1 mg/mL and the mixture was stirred for 1 hour at room temperature. The resulting solution was then subjected to a 100 kDa ultrafiltration membrane to remove the high-molecular-weight components. Since the resulting solution was alkaline, the solution was returned to neutral by dialysis. Finally, the solution was lyophilized to obtain a white solid. These products were designated SPG-S 1 to 4 and CUR-S1. Fig. 3 A shows an example of a GPC measurement for SPG-S3. The "before" represents SPG-S3a and the "after" represents SPG-S3. It is evident that the shoulder on the high-molecular-weight side has been removed.

GPC measurements in the Experimental Examples herein were carried out using a Prominence 501 system coupled with a Dawn-Heleos-A (Wyatt). 100 L of a solution containing 1.0 to 3.3 mg/mL of sample was injected into a system consisting of a Shodex HPLC pump (DU-H2130) equipped with a Shodex degassing unit (ERC-3125S). Chromatograms were obtained using a RI-501 interferometric differential refractive-index (RI) detector (Shodex) and a UV absorbance detector (SPD-20A; Shimadzu). These detectors were connected in tandem. For the measurement of polysaccharides, tandemly connected columns of SB-806MHQ and LB-803 in order from upstream to downstream were used. The mobile phase was 50 mM phosphate buffer containing 0.5 M KCl (pH 7.4) and ethylenediaminetetraacetic acid (EDTA) and the flow rate was 0.6 mL/min. For the measurement of complexes, tandemly connected columns of GF7M and GF510 were used and 50 mM phosphate buffer containing 0.5 M KCl and EDTA was used as the mobile phase. Here, EDTA was added to avoid unfavorable adsorption of phosphorothioate DNA by the GPC column.

[Experimental Example 2] Preparation and fractionation of complexes, and molecular weight determination

SPG obtained by lyophilization was added to 0.25N NaOH to a concentration of 100 μM and the solution was stirred for 1 hour at room temperature to obtain an SPG solution. The solutions were then mixed with a phosphate buffer, 100 μM nucleic acid solution (calculated assuming the whole oligonucleic acid molecule as one molecule) and SPG solution, in this order, and allowed to stand overnight at 4°C. The pH after mixing of these three solutions was 6 to 7. A comparison between before and after removal of the high-molecular-weight component is shown in Fig. 3B for SPG-S3a and SPG-S3. Here, the nucleic acid used was dA60, in which all phosphodiester bonds in the molecule were replaced by phosphorothioate bonds. In the Experimental Examples herein, dAx in which all of the phosphodiester bonds contained in the molecule have been replaced by phosphorothioate bonds may be abbreviated simply as "dAx". Although high-molecular-weight components were observed in the complex before removal (before), three peaks were clearly observed in the sample using SPG-S3 after removal and the high-molecular-weight components of the fraction around 20 minutes were completely removed.

Next, each of the peaks of the prepared complexes was isolated using the HPLC fraction collector. For example, eluates were collected at 24.3 to 24.6 min, 23.2 to 23.7 min and 21.8 to 22.3 min for the complexes prepared from SPG-S3 and dA60. They were designated q1, q2 and q3 from the low-molecular-weight side. This procedure was repeated to collect a sufficient volume of eluent, which was then lyophilized to obtain a white solid. Fig. 3C shows the GPC chromatograms of each of the samples of the three peaks obtained from the complex prepared from SPG-S3 and dA60. The peaks are unimodal, indicating that the isolation was successful. The molecular weight at the top of these peaks was then measured based on the light scattering. The results are shown in Table 1.

[Table 1]

| **dA60s** | **q 1** | **q 2** | **q 3** |
|---|---|---|---|
| **$M_W$(10$^4$g/mol)** | 5.04 | 10.3 | 15.3 |
| **Molecular weight ratio of each peak** | 1 | 2.04 | 3.02 |
| **Glucose number/ base number** | 1.8 | 2.2 | 2.3 |

The values obtained by dividing the molecular weights of q2 and q3 by q1 are indicated in the third column. These values are in the ratio of 1:2:3. From the obtained molecular weights, it was considered that q1 contains one molecule of dA60, q2 contains two molecules of dA60 and q3 contains three molecules of dA60. Based on this, the ratios of the mole number of main-chain glucoses to the mole number of bases were calculated. The results are shown in the fourth column. Hereafter, these complexes with low-molecular-weight and a stoichiometric ratio of dA to main-chain glucose of approximately 1:2 are described as a "low-molecular-weight complex". The low-molecular-weight complex containing one molecule of nucleic acid are described as q1, those containing two molecules as q2.

These results suggest the following: When dA60 is complexed with an SPG, the dA forms a complex with an SPG chain consistent with its own length from among various SPG chains with various lengths; this model may be called

DNA template model, as the DNA is considered to serve as a template (Fig. 4). When two dAs are present, they complex with a SPG chain of twice the length of those with a single dA. For this reason, it is considered that the molecular weight ratio of q1 and q2 is 2 and that the ratio of DNA to glucose contained in q1 and q2 is equal.

[Experimental Example 3] Formation of complexes of various lengths of phosphorothioate dA oligomers with SPG

In the same manner as in Experimental Example 2, complexes were formed from each of dA30, dA40 and dA60 with SPG-S3. The resulting complexes were separated by gel permeation chromatography, and each peak of the formed low-molecular-weight complexes (q1, q2, q3) was isolated to determine the molecular weight and other physical properties by light scattering spectrometer and other devices. The results are shown in Table 2.

[Table 2]

| | | $10^{-4}M_w$ | $M_w/M_n$ ($\pm$ 0.5$\sigma$) | qX/q1 | av. $\left(\frac{[mG]}{[dA]}\right)_f$ ($\pm$ 0.5$\sigma$) | $\langle S\rangle_z^{1/2}$ /nm | $10^{-4}M_w$ sSPG | $N_{mG}$[(b)] |
|---|---|---|---|---|---|---|---|---|
| | q1 | 2.51 $\pm$ 0.06 | 1.01 | - | - | -[(c)] | 0.77 | 35 (+5) |
| $dA_{30}$-SPG | q2 | 5.05 $\pm$ 0.07 | 1.02 | 2.01 | - | -[(c)] | 1.53 | 71 (+11) |
| | q3 | 7.69 $\pm$ 0.01 | 1.00 | 3.06 | - | 7.13 $\pm$ 1.8 | 2.37 | 109 (+19) |
| | q1 | 3.37 $\pm$ 0.01 | 1.01 | - | - | -[(c)] | 1.03 | 47 (+7) |
| $dA_{40}$-SPG | q2 | 6.59 $\pm$ 0.02 | 1.02 | 1.96 | - | -[(c)] | 1.98 | 91 (+11) |
| | q3 | 10.0 $\pm$ 0.05 | 1.00 | 2.97 | - | 9.23 $\pm$ 2.3 | 3.02 | 140 (+20) |
| | q1 | 4.95 $\pm$ 0.02 | 1.01 | 1.97[(a)] | 1.9 | 4.96 (SAXS) | 1.48 | 68 (+8) |
| $dA_{60}$-SPG | q2 | 9.90 $\pm$ 0.04 | 1.01 | 2.00 | 2.1 | 8.94 $\pm$ 1.6 | 2.98 | 137 (+17) |
| | q3 | 14.9 $\pm$ 0.04 | 1.02 | 3.01 | 2.2 | 12.5 $\pm$ 1.1 | 4.50 | 207 (+20) |

Table 2 shows physical properties of the fractionated low-molecular-weight complexes including their molecular weights and radii of gyration. (a) Mw of q1-dA60-SPG was divided by q1-dA30-SPG. (b) The number of main-chain glucoses calculated from Eq. 8 was indicated. The number in parentheses indicates the difference from the number of dAs. (c) SAXS was not performed because it was too small to be measured by SLS.

Surprisingly, the dispersion indices, expressed as Mw (weight-average molecular weight)/Mn (number-average molecular weight), were quite small compared to those of the original SPG, approximately 1.01 $\pm$ 0.01. Mw/Mn was measured with the analysis program provided by the light scattering instrument. The ratios of Mw of complexes made from the same dAx, e.g., the ratios obtained by dividing the Mws of q2-dA30-SPG and q3-dA30-SPG by the Mw of q1-dA30-SPG, were always 2 and 3, respectively. The calculated results are shown in the table as qx/q1. Even when the ratio of q1-dA30-SPG to q1-dA60-SPG was calculated, it was 2.0. These results suggest that dAx preferentially collects the most suitable SPGs in terms of the chain length when forming the complex. When one dAx molecule is contained, dAx collects only suitable short sSPG to form the complex of q1 (Fig. 4). When two dAx molecules are contained, they collect only sSPG that is twice as long as q1. When three dAx molecules are contained, the sSPG becomes three times as long as that of q1. The observed relation of qx/q1 suggests that such preferential selection occurs during the complexation process. This can be referred to as "dA-templated quantized complexation". Since the complex is known to exhibit a triple-helical structure formed from two SPG chains and one DNA chain, the number of main-chain glucoses ($N_{mG}$) can be calculated from the measured molecular weight of $M_{W.dAx\text{-}SPG}$ and the following formula.

$$N_{\mathrm{mG}} = 3\,\frac{1}{M_{\mathrm{SPG}}}\left(\frac{M_{\mathrm{w.dA_X SPG}} - N M_{\mathrm{w.dA_X}}}{2} - 18\right)$$

Here, $M_{SPG}$ is the molecular weight of the SPG repeating unit represented by the following chemical formula, i.e., 666.58.

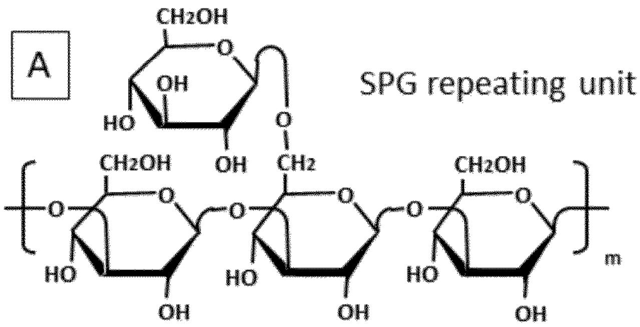

In addition, N is the number of dAx chains and $M_{W.dAx}$ is the molecular weight of dAx. The obtained $N_{mG}$ values are shown in the last column of the table. The molecular weight of one sSPG chain, i.e., $(M_{W.dAxSPG}-NM_{W\cdot dAx})/2$, is also shown. Surprisingly, the $N_{mG}$ values were almost identical to the number of dA units. In a previous report (Biomacromolecules 2001, 2 (3), 641-650), the stoichiometric ratio was determined to be [mG]/[dA] = 2.0, and this value was also supported by computer chemistry. The low-molecular-weight complexes of the present invention satisfy this stoichiometry.

[Experimental Example 4] Study of mixing ratio of dA60 and SPG-S-3

The molecular number ratio ([mG]/[dA])mix of dA60 and fractionated S-3 to be mixed was varied from 1.5 to 16 in Experimental Example 3 and RI and UV chromatograms were obtained. Samples were injected such that the amount of dA60 was fixed to be equal.

The results are shown in Fig. 5. When the ([mG]/[dA])mix was 1.5 and 2.0, uncomplexed dA60 was observed at the elution time(t) of 25.7 minutes. No uncomplexed dA60 was observed when the ([mG]/[dA])mix exceeded 4. Further increases in SPG resulted in simultaneous increases in the q1, q2, and q3 peaks only in the RI. There was no large difference between ([mG]/[dA])mix of 8 and 16. This is because UV detected only dA60, whereas RI detected both dA60 and SPG; the increase in RI with increasing ([mG]/[dA])mix as in 8 and 16 was due to an increase in uncomplexed SPG.

Several interesting phenomena are present in Fig. 5. First, in UV, an isosbestic point is observed at the elution time of 24.9 minutes (Fig. 5B). This provides evidence of one-to-one interconversion of reactants and products. Second, the peak positions of the q1, q2, and q3 do not change at all. This suggests that the molecular weights of these complexes do not change with increasing SPG. This is in marked contrast to complexes with larger SPGs, where a broad unimodal peak is observed and the position of the top of the peak shifts toward the larger molecular side as the ([mG]/[dA])mix is increased. Third, unreacted dA60 was present even in SPG-rich compositions, such as those with a ([mG]/[dA])mix of 4. This means that dA60 does not form a complex with sSPG if the length of the sSPG chain does not match dAx. This result is quite surprising because it suggests that dAx precisely selects only the suitable length of sSPGs to bind, and when it runs out, dAx no longer forms a complex with them. Fig. 5C shows that ([mG]/[dA])f is maintained at 2.0 for each fraction, as expected. Note that this excludes the case where ([mG]/[dA])mix is 16, since a large amount of unreacted SPG would lead to incorrect results.

[Experimental Example 5] Complex formation with dA60 depends on the kind of polysaccharide

In addition to SPG-S 1 to 4 and CUR-S 1, the same operation was performed for cellulose and pullulan (purchased from Fujifilm Wako) to examine whether the phenomenon shown in Fig. 3 is observed. The presence of complex formation was also examined by CD measurement, since DNA changes the conformation upon complexation, which is sensitively reflected in the circularly dichroism spectrum (CD). The results are shown in Table 3. The results suggest that this phenomenon is unique to β-1,3-glucan.

[Table 3]

| Sample name | Raw material β-1,3-glucan | Treatment for lowering molecular weight | Presence of complex formation |
|---|---|---|---|
| SPG-S1 | SPG<br>Mw = 1500K | 0.0 1 N NaOH<br>Ultrasonication | GPC CD<br>○ |
| SPG-S2 | SPG<br>Mw = 1500K | 2.5N alkali treatment | GPC CD<br>○ |

(continued)

| Sample name | Raw material $\beta$-1,3-glucan | Treatment for lowering molecular weight | Presence of complex formation |
|---|---|---|---|
| SPG-S3 | SPG<br>Mw = 800K | 0.01 N NaOH<br>Ultrasonication | GPC CD<br>○ |
| SPG-S4 | SPG<br>Mw = 800K | 2.5N alkali treatment | GPC CD<br>○ |
| CUR-S1 | Curdlan<br>FUJIFILM WAKO<br>4981481235458 | 2.5N alkali treatment | GPC CD<br>○ |
| CELL-S1 | Cellulose<br>FUJIFILM WAKO<br>4987481626829 | 2.5N alkali treatment | GPC CD<br>× |
| PULL-S1 | Pullulan<br>FUJIFILM WAKO<br>635-09071 | 2.5N alkali treatment | GPC CD<br>× |

[Experimental Example 6] Efficient production of low-molecular-weight complexes of dAx and SPG

30 mg of SPG with triple helix and molecular weight of 800,000 was dissolved in 30 mL of 0.25N NaOH (1 mg/mL) and stirred in a screw tube bottle for 1 hour to bring the SPG into a single-chain state. The resulting solution containing single-chain SPG was sonicated using a Qsonica Q700 ultrasonic homogenizer to lower the SPG in molecular weight. The ultrasonication conditions for one cycle are shown below.

Ultrasonication conditions

Amplitude 30 (ultrasonic output with Amplitude 100 as the maximum output of the device)
Pulse on time 1 min (ultrasonication time)
Pulse off time 3 min (ultrasonication-pause time)
Process time 30 min (total ultrasonication time)

Four cycles of the above conditions were performed so that the ultrasonication was applied for a total of 2 h. The SPG was lowered in molecular weight so that the molecular weight in the single-chain SPG state was reduced to be 50,000 to 30,000 or less. Heat treatment may be applied to accelerate the reaction.

The ultrasonicated sample was passed through a 0.45 μm Filter. The sample was subjected to ultrafiltration using an Amicon Ultra-0.5 Ultracel 50 ultrafiltration membrane (50 kDa). The infranatant liquid was dialyzed with a 1 kDa dialysis membrane for one day, and then freeze-dried in a lyophilizer for two days to obtain a solid. The resulting solid was again dissolved in 0.25N NaOH aqueous solution, stirred for 1 hour, and measured by GPC-MALS. The following system was used for GPC-MALS measurement of SPG.

Chromatography System
Pumping Unit: LC-20AD (SHIMADZU)
Autosampler: SIL-20A HT (SHIMADZU)
UV absorbance detector: SPD-20A (SHIMADZU)
Column Oven: CTO-20A (SHIMADZU)
Multi Angle Light Scattering Detector: Wyatt DAWN HELEOS-II (18-angle type) (Wyatt)
Differential Refractive Index Detector (Refractive Index Detector): Shodex RI-501 interferometric differential refractive-index

The measurement conditions are as follows.

Flow rate: 0.6 mL/min
Temperature: 40°C
Column: (for SPG measurement) SB-806M-HQ+SB-802.5M-HQ (Shodex)
Eluent: PBS + 10 mM EDTA
Injection volume: 100μL (sample concentration 1mg/mL)

The measurement results are shown in Fig. 6A. A majority of SPGs had molecular weights of 50,000 to 30,000 or less.

The SPG with lowered molecular weight and dA40s were mixed so that the ([mG]/[dA])mix was 8.0, and the complex was prepared according to the method in Experimental Example 2 and allowed to stand at 4°C overnight. The resulting complex was measured by GPC-MALS. The measurement conditions were as follows.

Flow rate: 0.6 mL/min
Temperature: 40°C
Column: GF-7M HQ+GF-510 (Shodex)
UV wavelength: 260 nm
Eluent: PBS + 10 mM EDTA
Injection volume: A sample equivalent to 7 μg of nucleic acid was injected.

The results are shown in Fig. 6B. q1 complex containing one molecule of nucleic acid in one molecule of complex was obtained at a yield of 80% or more by area ratio.

[Experimental Example 7] Formation of a complex of poly dA linked with antisense nucleic acid with low-molecular-weight SPG

A nucleic acid with the following sequence was used, in which an antisense sequence that suppresses the expression of YB-1 protein was added to the 5' end of $dA_{40}$. Hereinafter referred to as YB-1AS.

a^c^t^g^g^g^g^c^c^g^g^c^t^g^c^g^g^c^a^g^c^t^g^c^g^a^a^a^a^a^a^a^a^a
^a^a^a^a^a^a^a^a^a^a^a^a^a^a^a^a^a^a^a^a^a^a^a^a^a^a^a^a^a^a^a^a
(SEQ ID NO:1)

lower case letter: DNA ^: S modification

A low-molecular-weight complex was obtained from SPG-S3 and YB-1AS according to the method of Experimental Example 2. A chromatogram for the prefractionated state, in which q1, q2, and q3 are mixed, and chromatograms for q1, q2, and q3, obtained by isolating each peak, are shown in Fig. 7. The results suggest that even when an additional sequence is present at the end of the polydeoxyadenine (dAx), it forms a complex with an SPG chain which matches the length of the dAx from among SPG chains of various lengths, as in the case of polydeoxyadenine alone.

[Experimental Example 8] Physiological activity of complexes of antisense nucleic acid-linked poly(dA) and low-molecular-weight SPG

Human-derived lung cancer cells, PC9 cells, were seeded in 96-well plates at 1000 cells per well and incubated overnight at 37°C under 5% $CO_2$. Then, each complex sample was added to each well at a nucleic acid concentration of 0.4 μM and 1.0 μM, and the plate was incubated at 37°C under 5% $CO_2$ for 72h. After the incubation, cell viability was measured for each well. It has been shown that PC9 cells have a receptor for polysaccharide-nucleic acid complexes (Dectin-1), which allows them to selectively uptake nucleic acids (Cancer Gene Therapy volume 26, pages32-40(2019)).

The results are shown in Fig. 8. Surprisingly, the cell viability was reduced to a greater extent with the isolated low-molecular-weight complexes (q1, q2, and q3) compared to the polydisperse complexes or the mixture of low-molecular-weight complexes. The highest suppressing effect on cell viability was observed with q1. No reduction in cell viability was observed in A549 cells that did not express the receptor for the complex (Dectin-1).

[Experimental Example 9] Application to siRNA (miR-145)

A nucleic acid having the following sequence was used, in which siRNA (double-stranded) targeting human c-Myc was added to the 5' end of dA40. Hereafter, this nucleic acid is referred to as miR-145.

Sense

AGGGAUUCCUGGGAAAACUGGACGG^a^a^a^a^a^a^a^a^a^a^a^a^a^a^a^a^a^a^a^a^a^a^a^a^a^a^a^a^a^a^a^a^a^a^a^a^a^a^a^a (SEQ ID NO:2)

Antisense

G(M)U(F)C(M)C(F)A(M)G(F)U(M)U(F)U(M)U(F)C(M)C(F)C(M)A(F)G(M)G(F)A( M)A(F)U(M)C(F)C(M)C(F)U(M)^t^t (SEQ ID NO:3)

lower case letter: DNA upper case letter: RNA
^: S modification M: 2' OMe F: 2' F

Sense and antisense chains were hybridized, and low-molecular-weight complexes were prepared from SPG-S3 and miR-145 according to the method of Experimental Example 2, and q1, q2, and q3 were isolated according to Experimental Example 4. Subsequently, PC9 cells were seeded in 96-well plates at 1000 cells per well and incubated overnight at 37°C under 5% $CO_2$. Then, each complex sample was added to each well at 200 nM and 500 nM as nucleic acid concentration, and the cells were incubated at 37°C and 5% $CO_2$ for 72 h. After incubation, cell viability of each well was measured. As in Experimental Example 7, comparison was made in A549 cells.

The results are shown in Fig. 9. Even when double-stranded siRNA was linked to the 5' end of poly dA, cell viability was reduced to a greater extent when isolated low-molecular-weight complexes (q1, q2, q3) were used compared to polydisperse complexes or a mixture of small-molecular-size complexes. No reduction in cell viability was observed in A549 cells that did not express the receptor for the complex (Dectin-1).

[Experimental Example 10] Application to mismatch-containing microRNA (miR-143)

A nucleic acid having the following sequence was synthesized in which a microRNA (double-stranded) targeting human KRAS was added to the 5' end of dA40. Hereafter, this nucleic acid is referred to as miR-143. This nucleic acid has multiple mismatches in the RNA duplex region.

Sequence(5'→ 3')

Sense:UGAGGUGCAGUGCUGCAUCUCUGGa^a^a^a^a^a^a^a^a^a^a^a^a^a^a ^a^a^a^a^a^a^a^a^a^a^a^a^a^a^a^a^a^a^a^a^a^a^a^a^a (SEQ ID NO:4)

Antisense:U(F)^G(M)^A(F)G(M)A(F)U(M)G(F)A(M)A(F)G(M)C(F)A(M)C(F)U(M)G(F)U(M) A(F)G(M)C(F)U(M)C(F)A(M)^t^t (SEQ ID NO:5)

lower case letter: DNA upper case letter: RNA
^: S modification M: 2' OMe F: 2' F

Sense and antisense chains were hybridized, and low-molecular-weight complexes were prepared from SPG-S3 and miR-143 according to the method in Experimental Example 2, and q1, q2, and q3 were isolated according to Experimental Example 4. The effect of each complex to reduce cell viability was compared according to Experimental

Example 9.

The results are shown in Fig. 10. Even when a mismatch-containing double-stranded miRNA was linked to the 5' end of poly dA, cell viability was reduced to a greater extent when isolated low-molecular-weight complexes (q1, q2, q3) were used, compared to polydisperse complexes or a mixture of low-molecular-weight complexes. No reduction in cell viability was observed in A549 cells that did not express the receptor for the complex (Dectin-1).

[Experimental Example 11] Evaluation using other cells

Instead of PC-9 cells, human microglioma-derived cells, CCF-STTG1 cells, were used to evaluate the effect of miR-143 and miR-145 on cell viability according to Experimental Examples 9 and 10.

The results are shown in Fig. 11. Even with CCF -STTG 1 cells, cell viability was reduced to a greater extent when isolated low-molecular-weight complexes (q1, q2, q3) were used compared to polydisperse complexes or mixtures of low-molecular-weight complexes.

[Experimental Example 12] Confirmation of decreased expression of target proteins

PC-9 and A549 cells were seeded in 6-well plates at $1.0\times 10^5$ cells/ml and incubated at 37°C under 5% $CO_2$ for 24 hours. Then, complex samples were added, and cells were collected after 36h for YB-1 AS and after 72h for miR-145 and miR-143. The recovered cells were suspended in lysis buffer containing 10 mM Tris-HCl (pH 7.9), 150 mM NaCl, 0.5% and 1 mM PMSF, and ultrasonicated for 10 seconds. Cell lysates (protein amounts: YB-1: 10 μg, c-Myc: 50 μg, K-RAS: 30 μg) were separated on a 10% or 15% SDS-PAGE gel, and proteins were transferred to a PVDF membrane. The transferred proteins were then chemiluminated with antibodies. The results are shown in Fig. 12. It can be seen that the expression of each target protein was decreased.

[Experimental Example 13] Physiological activity of complex of poly dA linked with antisense nucleic acid and low-molecular-weight SPG

A nucleic acid with the following sequence was used, in which an antisense sequence that suppresses the expression of K-ras protein was added to the 5' end of $dA_{40}$. Hereinafter referred to as K-ras-AS.

c^g^g^g^a^g^t^a^c^t^g^g^c^c^g^a^g^c^c^g^c^c^g^c^c -dA40s (SEQ ID NO:6)

lower case letter: DNA ^: S modification

According to the method in Experimental Example 2, a low-molecular-weight complex was prepared from single-chain SPG and K-ras-AS, and q1 was isolated using the fraction connector of HPLC (q1-K-ras-AS). As a control, polydisperse complexes prepared from SPG that had not been lowered in molecular weight and K-ras-AS was used (L-SPG/K-ras-AS).

PC9 cells were seeded in 96-well plates at 1000 cells per well and incubated overnight at 37°C under 5% $CO_2$. Then, each complex sample was added to each well at 0.4μM and 1.0μM as nucleic acid concentration, and incubated at 37°C under 5% $CO_2$ for 72 hours. After incubation, cell viability was measured by WST-8-assay for each well. The results are shown in Fig. 13. The cell viability was reduced to a greater extent when the isolated low-molecular-weight complex (q1-K-ras-AS) was used as compared to polydisperse complexes.

[Experimental Example 14] Physiological activity of the complex of poly dA linked with antisense nucleic acid and low-molecular-weight SPG

PC-9 cells were seeded in 6-well plates at $1.0\times 10^5$ cells/ml and incubated at 37°C under 5% $CO_2$ for 24 hours. Then, the complex samples prepared in Experimental Example 13 (L-SPG/K-ras-AS and q1-K-ras-AS) were added and the cells were collected after 72 hours. The collected cells were suspended in lysis buffer containing 10 mM Tris-HCl (pH 7.9), 150 mM NaCl, 0.5% NP40 and 1 mM PMSF and ultrasonicated for 10 seconds. Cell lysate (protein content: 30 μg) was separated on a 15% SDS-PAGE gel, and the protein was transferred to a PVDF membrane. K-ras protein was then detected by Western blotting using an antibody against K-ras. The results are shown in Fig. 14. The expression of K-ras protein was reduced to a greater extent when the isolated low-molecular-weight complex (q1-K-ras-AS) was used as compared to the polydisperse complexes.

[Experimental Example 15] Physiological activity of a complex of poly dA linked with CPG DNA and low-molecular-weight SPG

A nucleic acid having the following sequence was used, in which the CpG sequence was added to the 5' end of $dA_{40}$. Hereinafter referred to as K3.

a^t^c^g^a^c^t^c^t^c^g^a^g^c^g^t^t^c^t^c+dA40s (SEQ ID NO:7)

lower case letter: DNA ^: S modification

Low-molecular-weight complexes were prepared from single-chain SPG and K3 according to the method in Experimental Example 2, and q1, q2, and q3 were isolated using HPLC fraction connectors (q1-K3, q2-K3, q3-K3). As controls, polydisperse complexes prepared from SPG that had not been lowered in molecular weight and K3 (L-SPG/K3) and K3 without forming a complex with SPG (naked K3) were used.

Splenocytes were collected from 6-week-old male C57BL/6JJmsSlc mice and seeded to $1.0\times10^6$ cells in 96-well plates. In addition, each complex was added at 20 nM of the nucleic acid concentration and the cells were incubated for 24h. After that, the culture medium was collected to determine the IL-6 concentration by ELISA. The results are shown in Fig. 15. Unlike antisense nucleic acids and miRNAs, in the case of CpG DNA, the q1 complex (q1-K3) induced rather lower IL-6 production as compared to the polydisperse complexes (L-SPG/K3) and showed values comparable to those of naked CpG DNA.

INDUSTRIAL APPLICABILITY

A chemically homogeneous population of β-1,3-glucan/nucleic acid complexes is provided by the present invention. The population of β-1,3-glucan/nucleic acid complexes of the present invention is homogeneous in terms of the number of nucleic acid molecules contained in one molecule of the complex, the size of the β-1,3-glucan expressed as the mole number of main-chain glucoses, the size of the complex or the like, and is advantageous for development as a medicine in view of chemical "assurance of equivalence". The population of β-1,3-glucan/nucleic acid complexes of the present invention also has a superior capability for delivering a nucleic acid and is useful as a carrier for introducing a nucleic acid into a cell. The present invention also provides a population of β-1,3-glucan/nucleic acid complexes which is abundant in low-molecular-weight β-1,3-glucan/nucleic acid complexes that are of superior capability for delivering a nucleic acid.

This application is based on a patent application No. 2020-171532 filed in Japan (filing date: October 9, 2020), the contents of which are incorporated in full herein.

**Claims**

1. A population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising the homo-polynucleotide that binds to β-1,3-glucan via a hydrogen bond contained in the nucleic acid,
   wherein 30% or more of the β-1,3-glucan/nucleic acid complexes contained in the population satisfy the following conditions:

   (i) they are homogeneous in the length of the homo-polynucleotide contained in the nucleic acid comprising a homo-polynucleotide that bind to β-1,3-glucan via a hydrogen bond constituting the complex, and
   (ii-a) the molecular number of the nucleic acid(s) comprising a homo-polynucleotide that binds to β-1,3-glucan via a hydrogen bond contained in one molecule of the complex is 1, 2 or 3.

2. The population of the β-1,3-glucan/nucleic acid complexes of claim 1, wherein 40% or more of the β-1,3-glucan/nucleic acid complexes contained in the population satisfy the conditions (i) and (ii-a).

3. The population of the β-1,3-glucan/nucleic acid complexes of claim 1, wherein 50% or more of the β-1,3-glucan/nucleic acid complexes contained in the population satisfy the conditions (i) and (ii-a).

**4.** The population of the P-1,3-glucan/nucleic acid complexes of claim 1, wherein 60% or more of the P-1,3-glucan/nucleic acid complexes contained in the population satisfy the conditions (i) and (ii-a).

**5.** The population of the β-1,3-glucan/nucleic acid complexes of claim 1, wherein 70% or more of the β-1,3-glucan/nucleic acid complexes contained in the population satisfy the conditions (i) and (ii-a).

**6.** The population of the β-1,3-glucan/nucleic acid complexes of claim 1, wherein 80% or more of the β-1,3-glucan/nucleic acid complexes contained in the population satisfy the conditions (i) and (ii-a).

**7.** The population of the β-1,3-glucan/nucleic acid complexes of claim 1, wherein 90% or more of the β-1,3-glucan/nucleic acid complexes contained in the population satisfy the conditions (i) and (ii-a).

**8.** The population of the P-1,3-glucan/nucleic acid complexes of any one of claims 1 to 7, wherein the molecular number of the nucleic acid(s) comprising a homo-polynucleotide that binds to β-1,3-glucan via a hydrogen bond contained in one molecule of the complex as defined in (ii-a) is 1 or 2.

**9.** The population of the β-1,3-glucan/nucleic acid complexes of any one of claims 1 to 8, wherein 50% or more of the total number of the β-1,3-glucan/nucleic acid complexes satisfying the conditions (i) and (ii-a) are a β-1,3-glucan/nucleic acid complex in which the molecular number of the nucleic acid(s) comprising a homo-polynucleotide that binds to β-1,3-glucan via a hydrogen bond contained in one molecule of the complex is 1.

**10.** A population of β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein 30% or more of the β-1,3-glucan/nucleic acid complexes contained in the population satisfy the following conditions:

(i) they are homogeneous in the length of the homo-polynucleotide contained in the nucleic acid comprising a homo-polynucleotide that bind to β-1,3-glucan via a hydrogen bond constituting the complex, and
(ii-b) they are homogeneous in the molecular number of the nucleic acid (s) comprising a homo-polynucleotide that binds to β-1,3-glucan via a hydrogen bond contained in one molecule of the complex, wherein the molecular number of the nucleic acid(s) is 1, 2 or 3.

**11.** The population of the β-1,3-glucan/nucleic acid complexes of claim 10, wherein 40% or more of the β-1,3-glucan/nucleic acid complexes contained in the population satisfy the conditions (i) and (ii-b).

**12.** The population of the β-1,3-glucan/nucleic acid complexes of claim 10, wherein 50% or more of the β-1,3-glucan/nucleic acid complexes contained in the population satisfy the conditions (i) and (ii-b).

**13.** The population of the β-1,3-glucan/nucleic acid complexes of claim 10, wherein 60% or more of the β-1,3-glucan/nucleic acid complexes contained in the population satisfy the conditions (i) and (ii-b).

**14.** The population of the β-1,3-glucan/nucleic acid complexes of claim 10, wherein 70% or more of the β-1,3-glucan/nucleic acid complexes contained in the population satisfy the conditions (i) and (ii-b).

**15.** The population of the β-1,3-glucan/nucleic acid complexes of claim 10, wherein 80% or more of the β-1,3-glucan/nucleic acid complexes contained in the population satisfy the conditions (i) and (ii-b).

**16.** The population of the β-1,3-glucan/nucleic acid complexes of claim 10, wherein 90% or more of the β-1,3-glucan/nucleic acid complexes contained in the population satisfy the conditions (i) and (ii-b).

**17.** The population of the β-1,3-glucan/nucleic acid complexes of any one of claims 10 to 16, wherein the molecular number of the nucleic acid(s) comprising a homo-polynucleotide that binds to β-1,3-glucan via a hydrogen bond contained in one molecule of the complex as defined in (ii-b) is 1.

**18.** The population of the β-1,3-glucan/nucleic acid complexes of any one of claims 1 to 17, wherein the average ratio of the number of main-chain glucose units of the β-1,3-glucan to the number of nucleotide units constituting the

homo-polynucleotide (number of main-chain glucose units/number of nucleotide units) contained in one molecule of the complex is 1.5 to 2.5.

**19.** The population of the β-1,3-glucan/nucleic acid complexes of any one of claims 1 to 18, wherein 30% or more of the β-1,3-glucan/nucleic acid complexes contained in the population further satisfy the following condition:
(iii) the molecular number of β-1 ,3-glucan(s) contained in one molecule of the complex is 1 or 2.

**20.** The population of the β-1,3-glucan/nucleic acid complexes of claim 19, wherein 80% or more of the β-1,3-glucan/nucleic acid complexes contained in the population further satisfy the condition (iii).

**21.** The population of the β-1,3-glucan/nucleic acid complexes of any one of claims 1 to 20, wherein the length of the homo-polynucleotide contained in the nucleic acid comprising a homo-polynucleotide that bind to β-1,3-glucan via a hydrogen bond constituting the complex as defined in (i) is 20 to 100 bases.

**22.** The population of the β-1,3-glucan/nucleic acid complexes of any one of claims 1 to 21, wherein the homo-polynucleotide is polydeoxyadenylic acid.

**23.** The population of the β-1,3-glucan/nucleic acid complexes of any one of claims 1 to 22, wherein the β-1,3-glucan is schizophyllan or curdlan.

**24.** The population of the β-1,3-glucan/nucleic acid complexes of claim 23, wherein the β-1,3-glucan is schizophyllan.

**25.** The population of the β-1,3-glucan/nucleic acid complexes of any one of claims 1 to 24, wherein the nucleic acid comprising the homo-polynucleotide that bind to β-1,3-glucan via a hydrogen bond comprises an additional nucleic acid, and wherein the additional nucleic acid is linked to the end of the homo-polynucleotide.

**26.** The population of the β-1,3-glucan/nucleic acid complexes of claim 25, wherein the additional nucleic acid is linked to the 5' end of the homo-polynucleotide.

**27.** The population of the β-1,3-glucan/nucleic acid complexes of claim 25 or 26, wherein the additional nucleic acid has an expression suppressing activity against a target gene.

**28.** The population of the β-1,3-glucan/nucleic acid complexes of any one of claims 25 to 27, wherein the additional nucleic acid is an antisense nucleic acid, siRNA or miRNA.

**29.** A production method of the population of the β-1,3-glucan/nucleic acid complexes of any one of claims 1 to 28, which comprises

a step of mixing a nucleic acid comprising a homo-polynucleotide that binds to β-1,3-glucan via a hydrogen bond with β-1,3-glucan to prepare β-1,3-glucan/nucleic acid complexes, each of which comprises β-1,3-glucan and a nucleic acid comprising a homo-polynucleotide that binds to β-1,3-glucan via a hydrogen bond, and has a triple-helical structure consisting of two β-1,3-glucan chains and one nucleic acid chain comprising a homo-polynucleotide that binds to β-1,3-glucan via a hydrogen bond contained in the nucleic acid,
wherein the β-1,3-glucan provided for mixing has a weight-average molecular weight of 100,000 or less and has a peak area for β-1,3-glucan with a molecular weight of 100,000 or more accounting for 50% or less of the total when separated by gel permeation chromatography and detected with a differential refractive index detector.

**30.** The production method of claim 29, wherein the ratio of the total number of main-chain glucose units contained in β-1,3-glucan provided for mixing to the total number of nucleotide units constituting the homo-polynucleotide contained in the nucleic acid provided for mixing comprising a homo-polynucleotide that binds to β-1,3-glucan via a hydrogen bond (the total number of main-chain glucose units/the total number of nucleotide units) is 4 or more.

**31.** The production method of claim 30, wherein the total number of main-chain glucose units/the total number of nucleotide units is 16 or less.

**32.** The production method of any one of claims 29 to 31, which further comprises a step of subjecting the resulting β-1,3-glucan/nucleic acid complexes to gel permeation chromatography to isolate a β-1,3-glucan/nucleic acid complex in which the molecular number of the nucleic acid(s) comprising a homo-polynucleotide that binds to β-1,3-glucan

via a hydrogen bond contained in one molecule of the complex is selected from the group consisting of 1, 2 and 3.

**33.** A composition for introducing a nucleic acid into a cell, which comprises the population of the β-1,3-glucan/nucleic acid complexes of any one of claims 1 to 28 to [28].

**34.** The composition of claim 33, wherein the cell is a Dectin-1 positive cell.

**35.** A method for introducing a nucleic acid into a cell, which comprises bringing the population of the β-1,3-glucan/nucleic acid complexes of any one of claims 1 to 28 into contact with the cell.

**36.** Use of the population of the β-1,3-glucan/nucleic acid complexes of any one of claims 1 to 28, for introducing a nucleic acid into a cell.

**37.** A composition for suppressing an expression of a target gene in a cell, which comprises the population of the β-1,3-glucan/nucleic acid complexes of claim 27 or 28.

**38.** The composition of claim 37, wherein the cell is a Dectin-1 positive cell.

**39.** A method for suppressing an expression of a target gene in a cell, which comprises bringing the population of the β-1,3-glucan/nucleic acid complexes of claim 27 or 28 into contact with the cell.

**40.** Use of the population of the β-1,3-glucan/nucleic acid complexes of claim 27 or 28, for suppressing an expression of a target gene in a cell.

FIG. 1

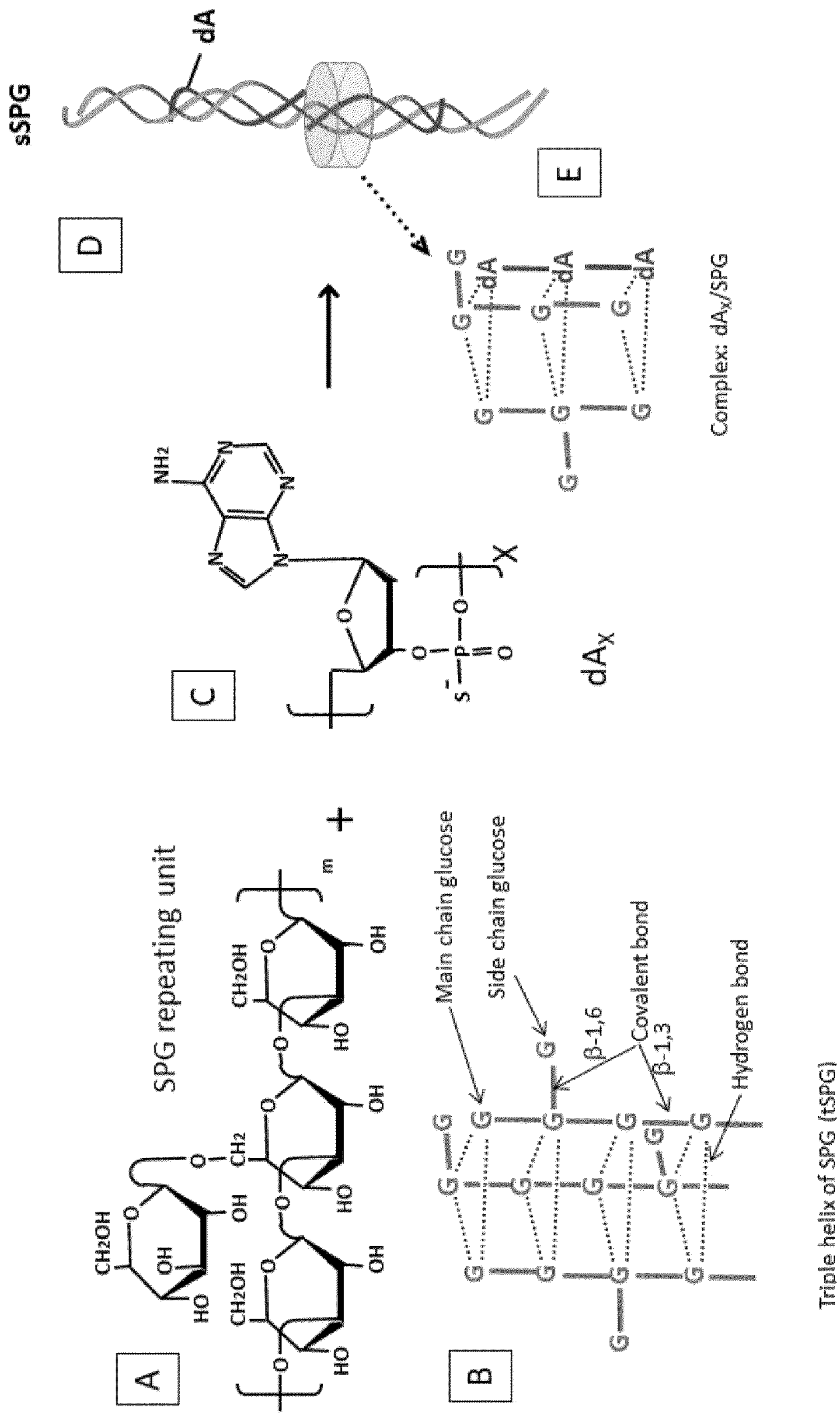
A
SPG repeating unit
B
Main chain glucose
Side chain glucose
Covalent bond
β-1,6
β-1,3
Hydrogen bond
Triple helix of SPG (tSPG)
C
$dA_X$
D
sSPG
dA
E
Complex: $dA_X$/SPG

FIG. 2

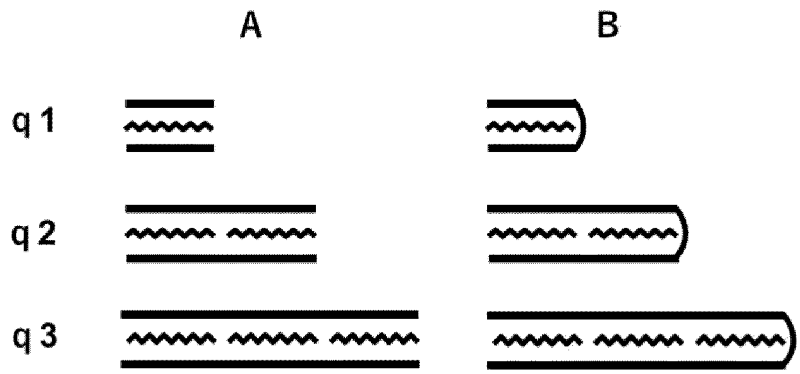

FIG. 3

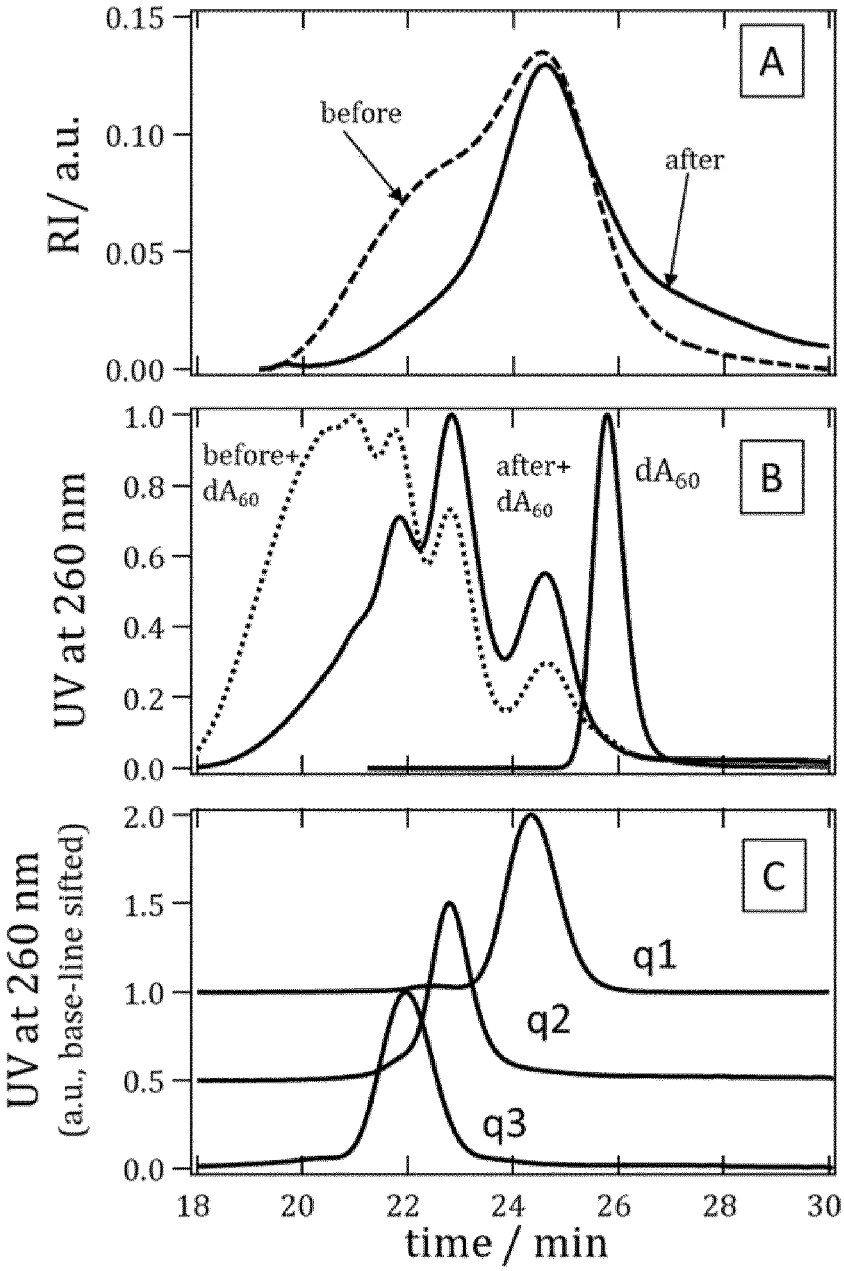
A
RI / a.u.
0.15
0.10
0.05
0.00
before
after
B
UV at 260 nm
1.0
0.8
0.6
0.4
0.2
0.0
before+ dA60
after+ dA60
dA60
C
UV at 260 nm (a.u., base-line sifted)
2.0
1.5
1.0
0.5
0.0
q1
q2
q3
18
20
22
24
26
28
30
time / min

FIG. 4

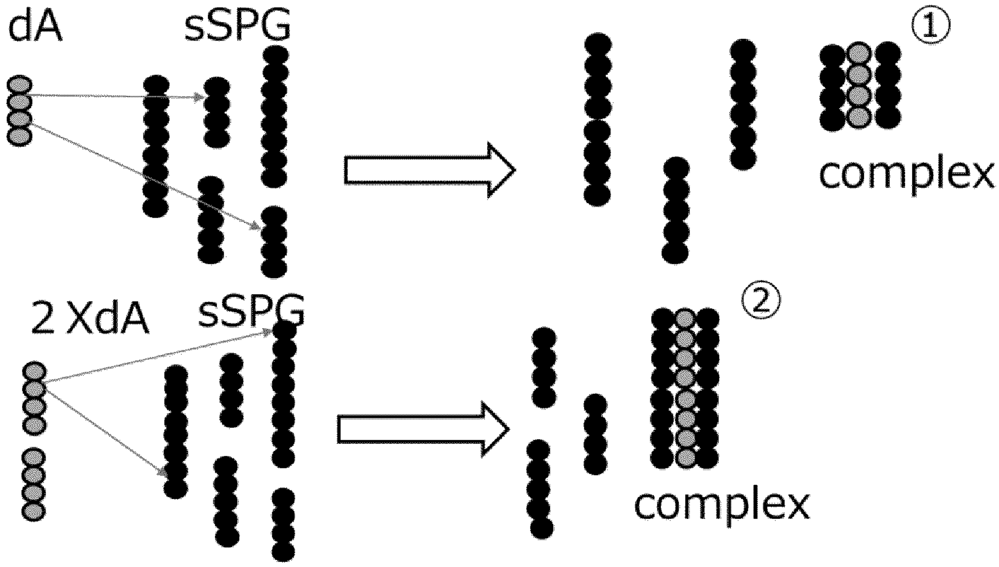

FIG. 5

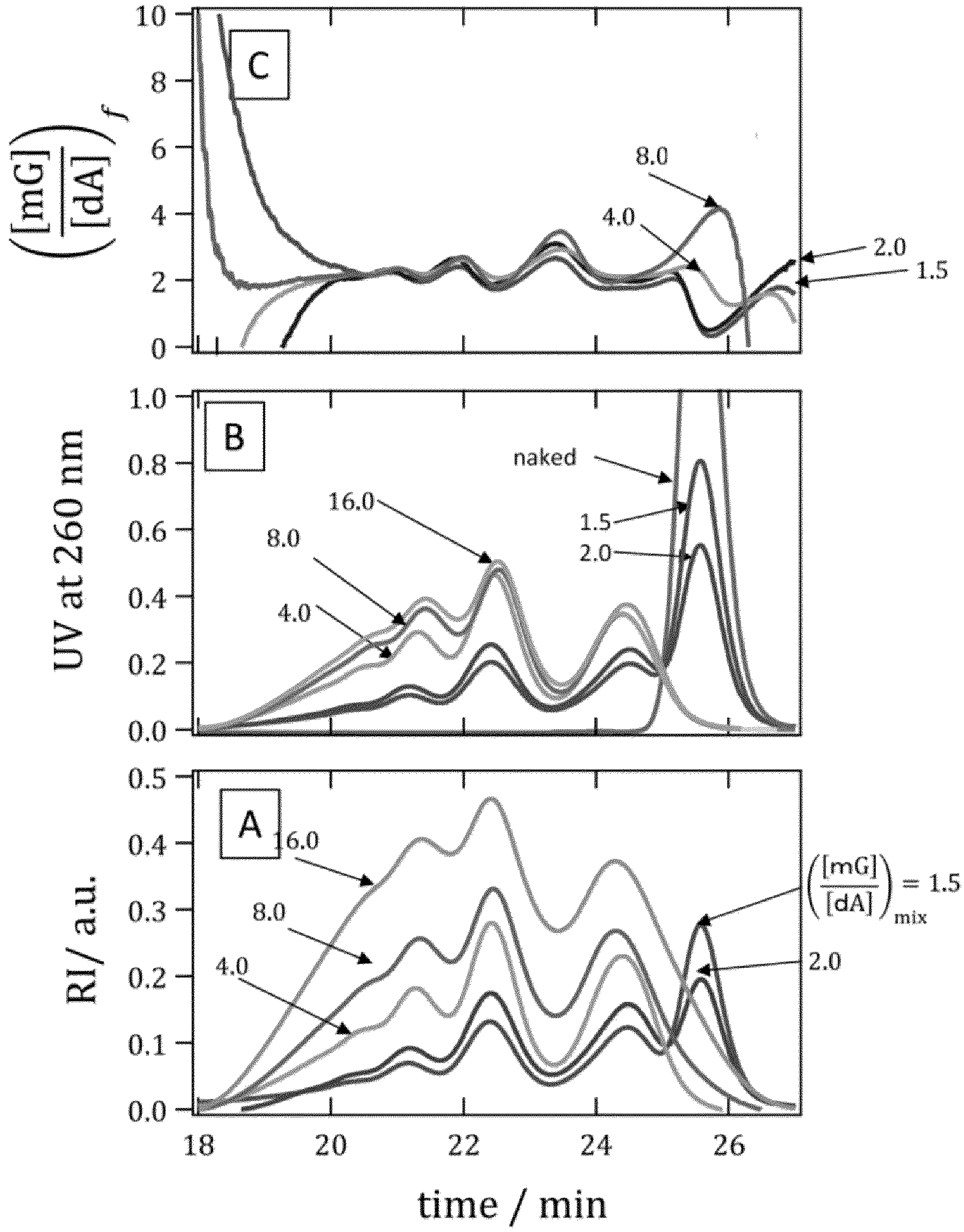
C
8.0
4.0
2.0
1.5
B
naked
16.0
8.0
4.0
1.5
2.0
UV at 260 nm
A
16.0
8.0
4.0
RI/ a.u.
([mG]/[dA])mix = 1.5
2.0
time / min

FIG. 6

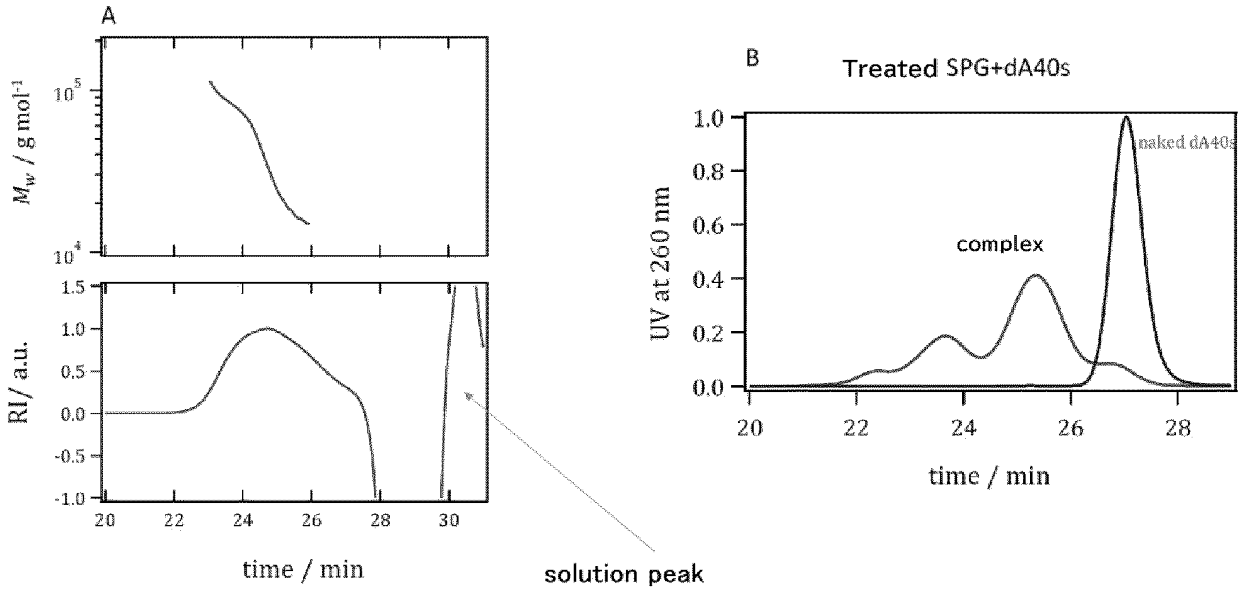
A
$M_w$ / g mol$^{-1}$
$10^5$
$10^4$
RI / a.u.
1.5
1.0
0.5
0.0
-0.5
-1.0
20
22
24
26
28
30
time / min
solution peak
B
Treated SPG+dA40s
UV at 260 nm
1.0
0.8
0.6
0.4
0.2
0.0
naked dA40s
complex
20
22
24
26
28
time / min

# FIG. 7

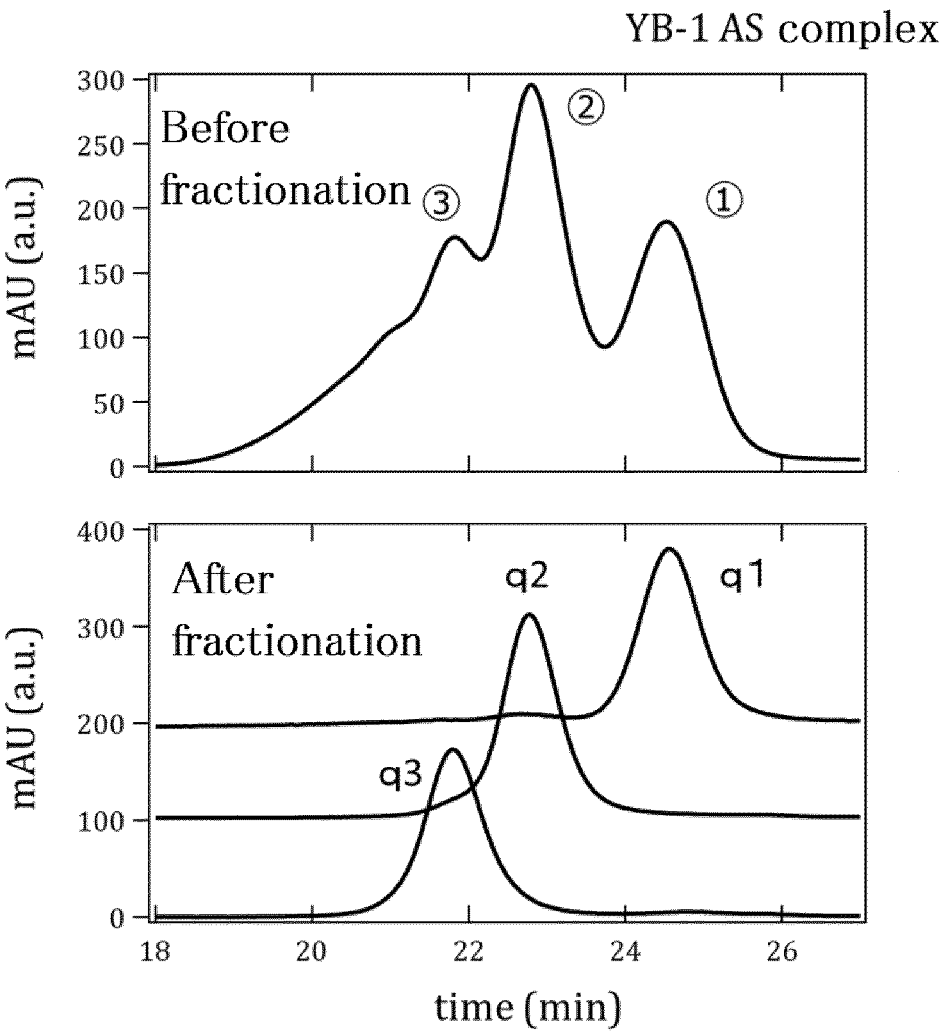
YB-1 AS complex
Before
fractionation
①
②
③
mAU (a.u.)
300
250
200
150
100
50
0
After
fractionation
q1
q2
q3
400
300
200
100
0
18
20
22
24
26
time (min)

# FIG. 8

PC-9

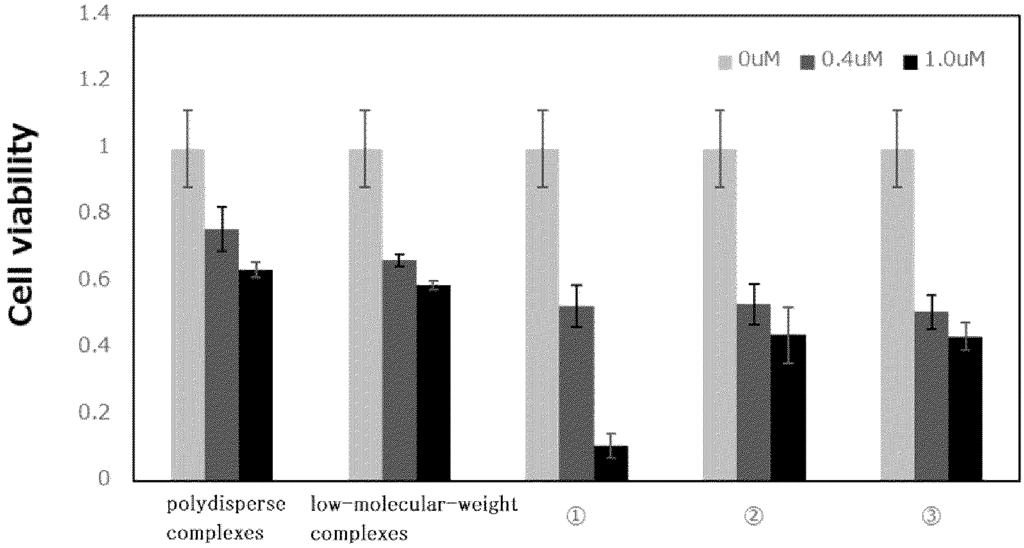


A549

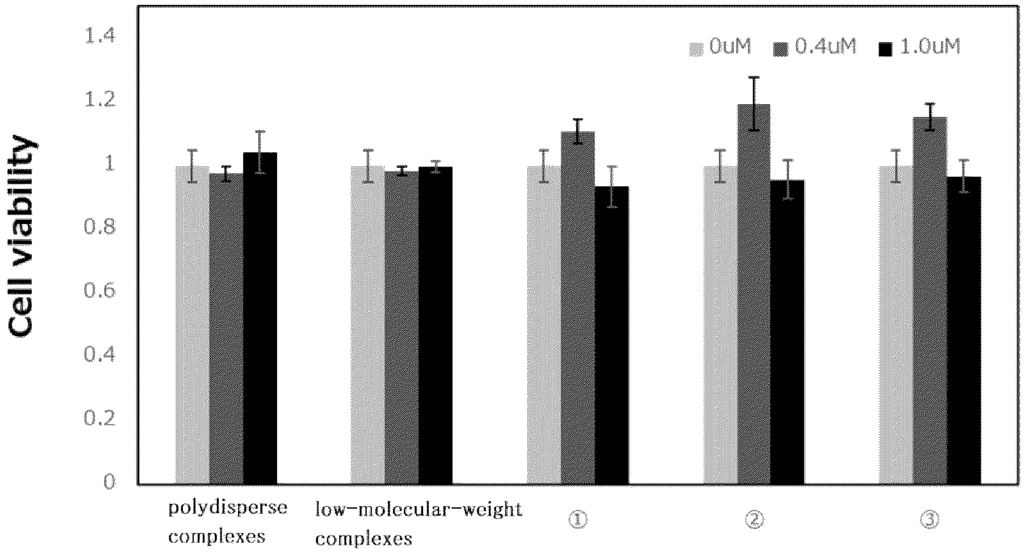

# FIG. 9

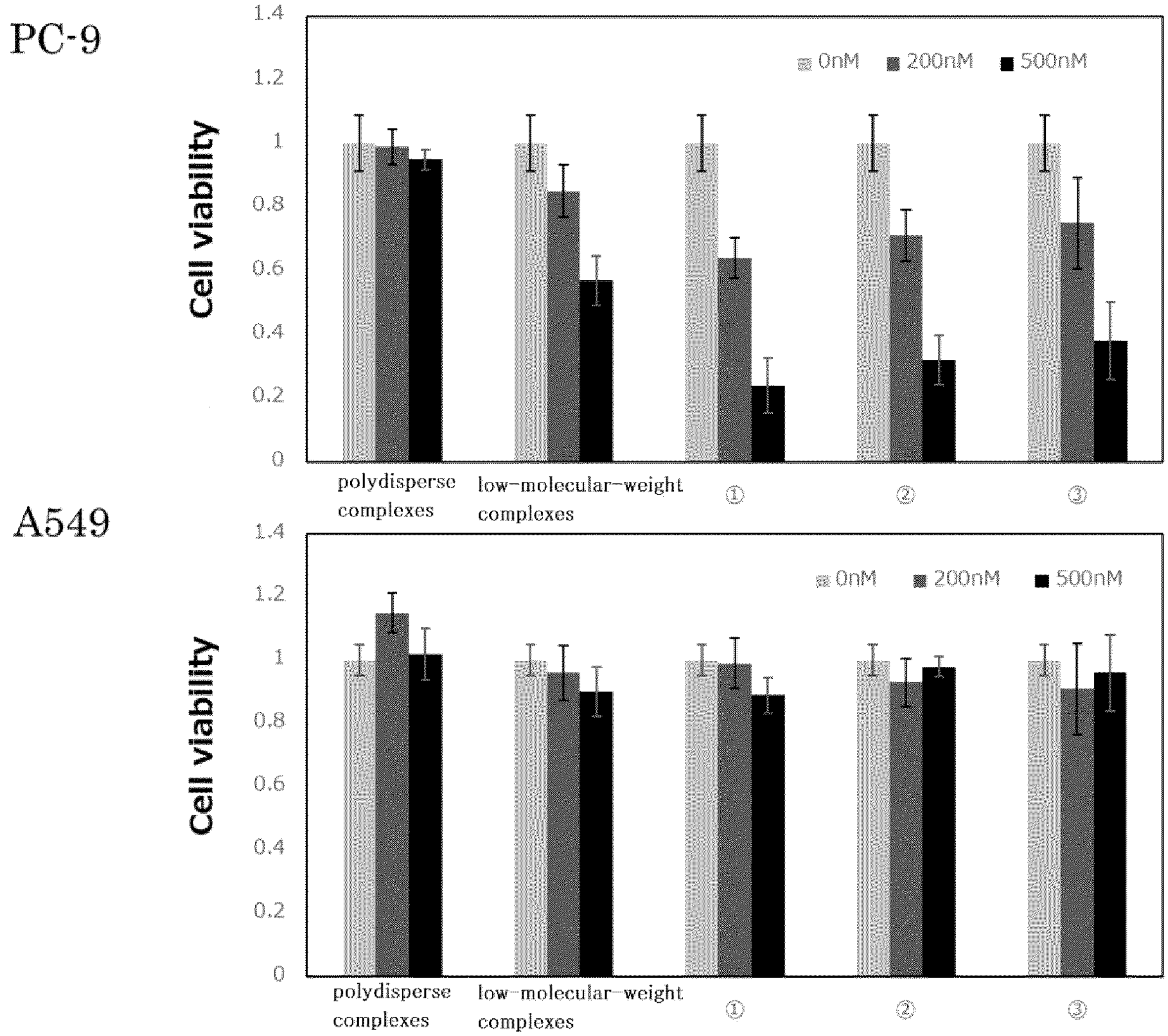
PC-9
Cell viability
1.4
1.2
1
0.8
0.6
0.4
0.2
0
0nM
200nM
500nM
polydisperse complexes
low-molecular-weight complexes
①
②
③
A549
Cell viability
1.4
1.2
1
0.8
0.6
0.4
0.2
0
0nM
200nM
500nM
polydisperse complexes
low-molecular-weight complexes
①
②
③

FIG. 10

PC-9

A549

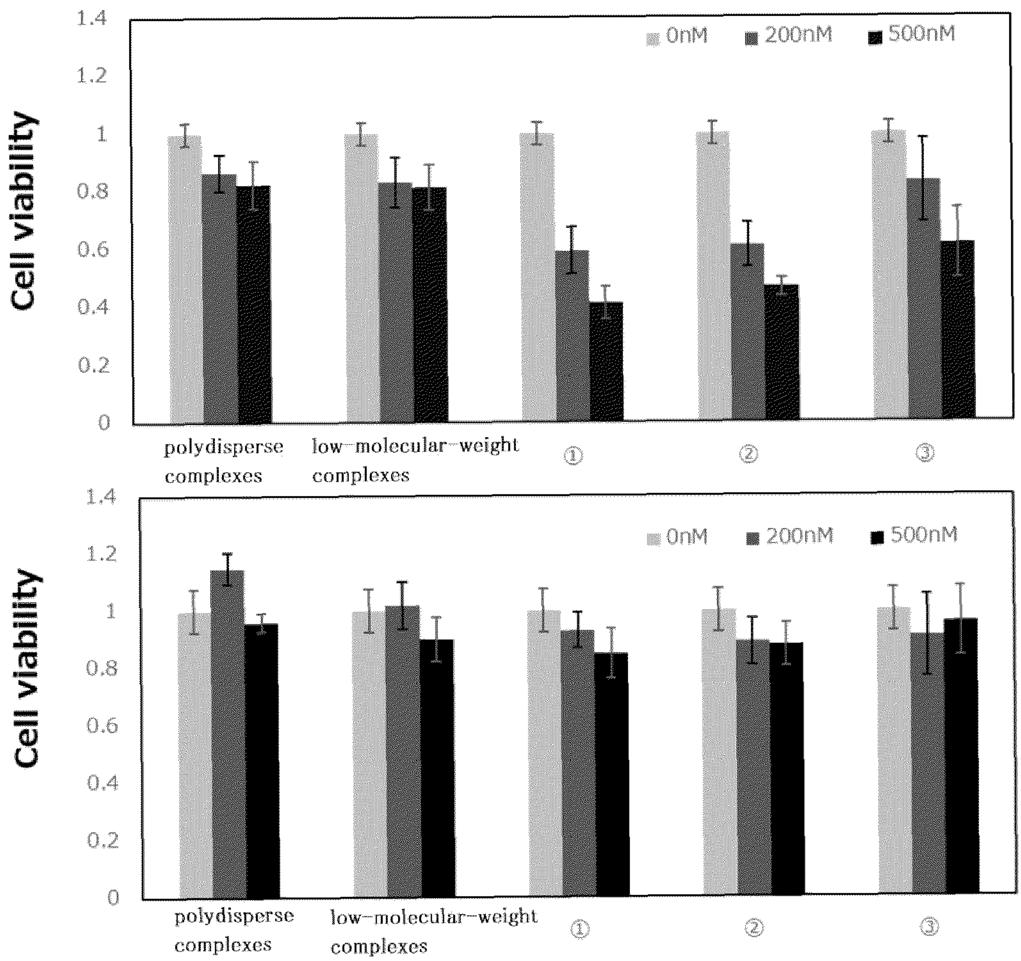

FIG. 11

miR-145

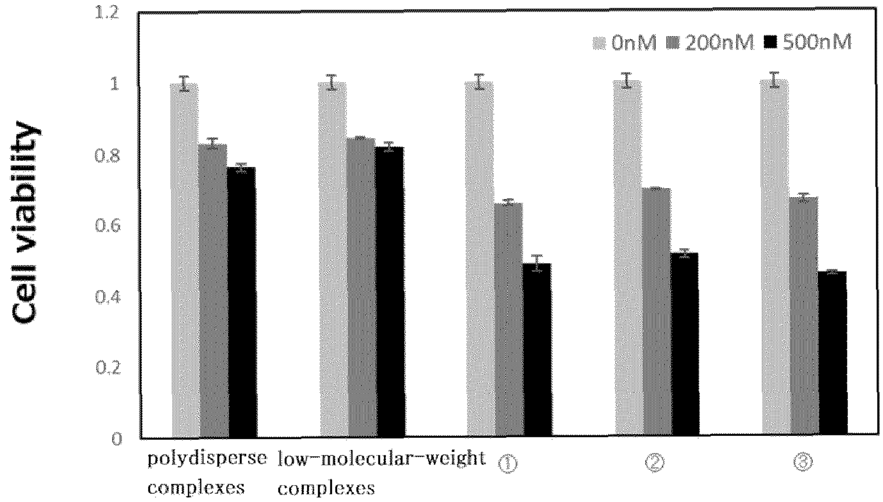


miR-143

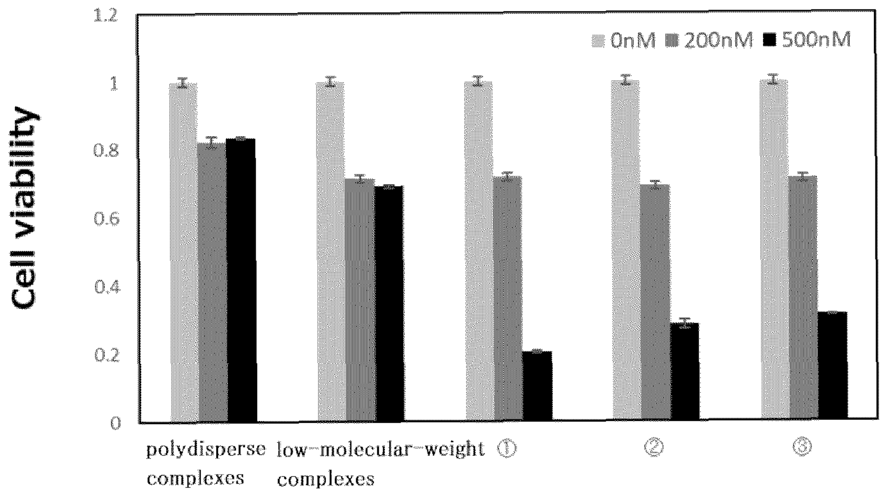

# FIG. 12

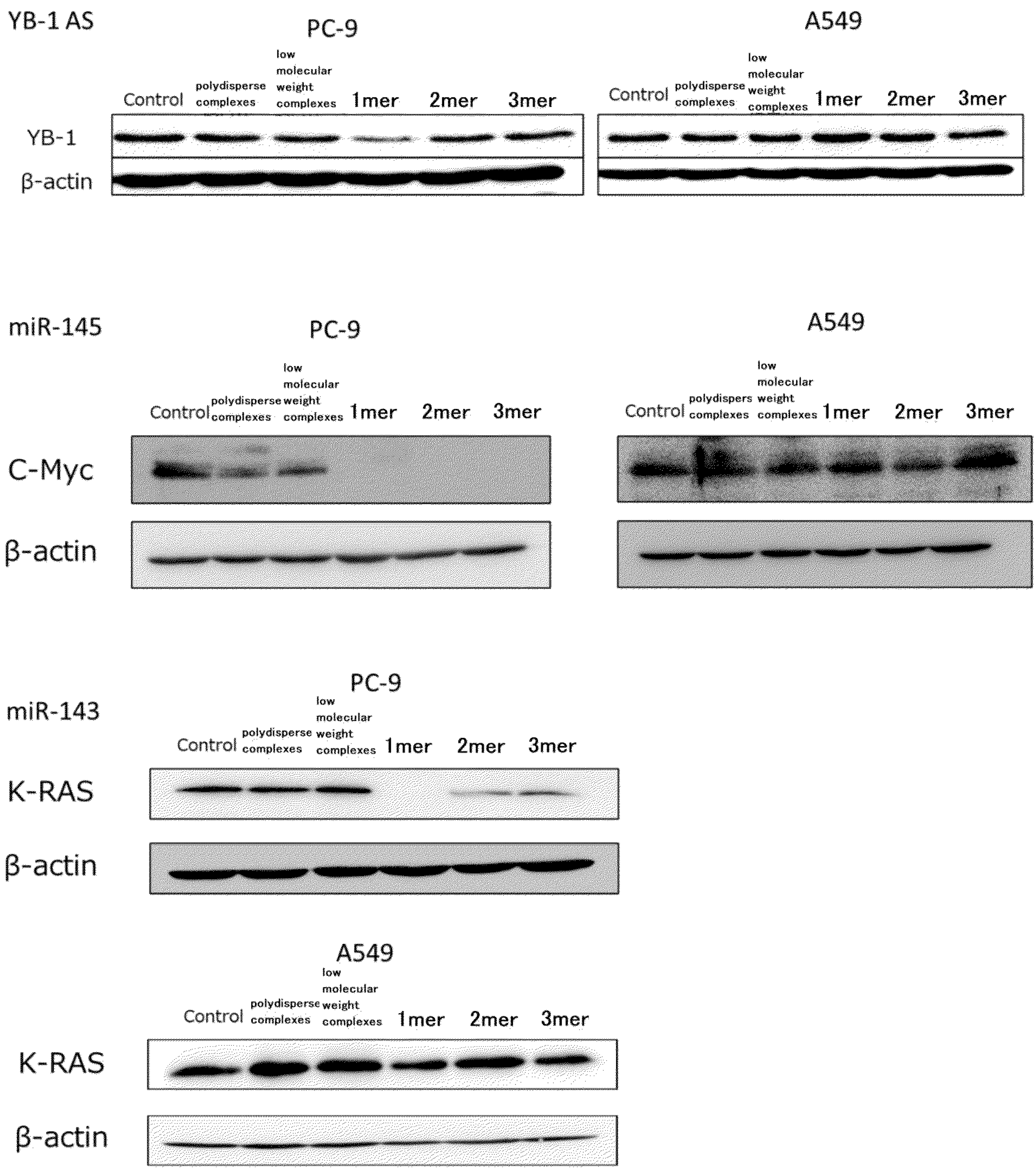
YB-1 AS
PC-9
A549
Control
polydisperse complexes
low molecular weight complexes
1mer
2mer
3mer
YB-1
β-actin
miR-145
C-Myc
miR-143
K-RAS

FIG. 13

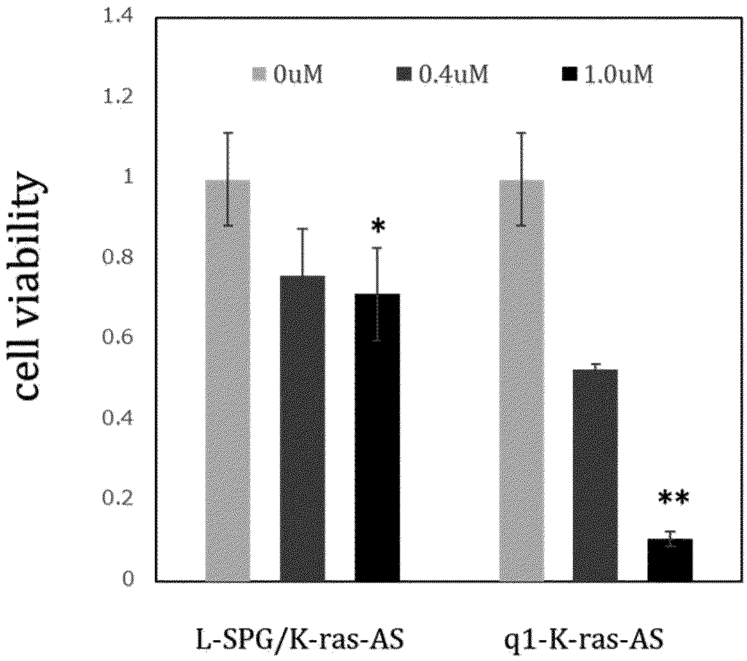
1.4
1.2
1
0.8
0.6
0.4
0.2
0
cell viability
0uM
0.4uM
1.0uM
*
**
L-SPG/K-ras-AS
q1-K-ras-AS

FIG. 14

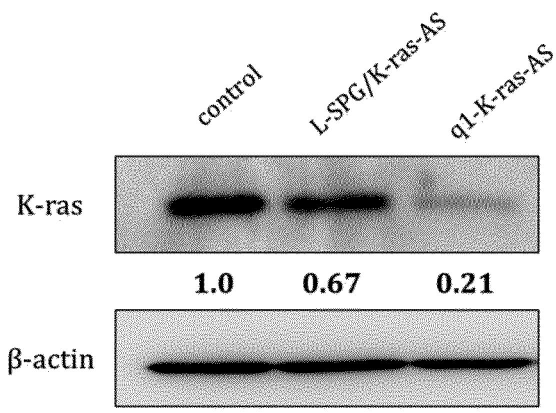
control
L-SPG/K-ras-AS
q1-K-ras-AS
K-ras
1.0
0.67
0.21
β-actin

FIG. 15

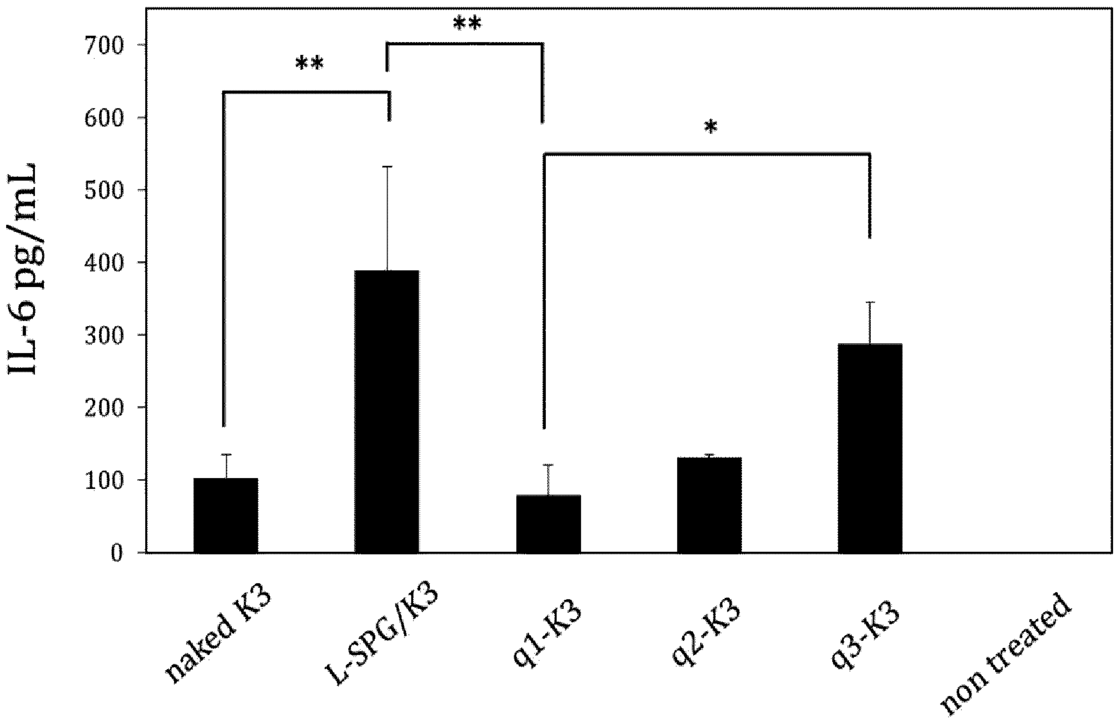
IL-6 pg/mL
700
600
500
400
300
200
100
0
**
**
*
naked K3
L-SPG/K3
q1-K3
q2-K3
q3-K3
non treated

**INTERNATIONAL SEARCH REPORT**

International application No.
**PCT/JP2021/037413**

**A. CLASSIFICATION OF SUBJECT MATTER**

***C12N 15/87***(2006.01)i; ***A61K 31/7088***(2006.01)i; ***A61K 31/7105***(2006.01)i; ***A61K 31/713***(2006.01)i; ***A61K 47/61***(2017.01)i; ***A61K 48/00***(2006.01)i; ***A61P 31/10***(2006.01)i; ***C12N 15/113***(2010.01)i
FI: C12N15/87 Z ZNA; A61K31/7088; A61K31/7105; A61K31/713; A61K47/61; A61K48/00; A61P31/10; C12N15/113 Z

According to International Patent Classification (IPC) or to both national classification and IPC

**B. FIELDS SEARCHED**

Minimum documentation searched (classification system followed by classification symbols)

C12N15/00-15/90; A61K31/7088; A61K31/7105; A61K31/713; A61K47/61; A61K48/00; A61P31/10

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN); Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | WO 2016/098832 A1 (PUBLIC UNIVERSITY CORP THE UNIVERSITY OF KITAKYUSHU) 23 June 2016 (2016-06-23)<br>claims, paragraphs [0004]-[0007], [0031]-[0059], examples | 1-26, 29, 32-33, 35-36 |
| Y | claims, paragraphs [0004]-[0007], [0031]-[0059], examples | 27-40 |
| Y | JP 2010-207109 A (NISHIHIRA, Jun) 24 September 2010 (2010-09-24)<br>claims, paragraphs [0017]-[0018], [0033]-[0040], examples | 27-40 |
| A | JP 2011-178707 A (JAPAN SCIENCE & TECHNOLOGY AGENCY) 15 September 2011 (2011-09-15)<br>see entire text | 1-40 |
| A | WO 2018/097296 A1 (NAPAJEN PHARMA INC) 31 May 2018 (2018-05-31)<br>see entire text | 1-40 |
| A | JP 2008-100919 A (JAPAN SCIENCE & TECHNOLOGY AGENCY) 01 May 2008 (2008-05-01)<br>see entire text | 1-40 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 December 2021** | **21 December 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/037413**

**C. DOCUMENTS CONSIDERED TO BE RELEVANT**

| Category* | Citation of document, with indication, where appropriate, of the relevant passages | Relevant to claim No. |
|---|---|---|
| A | MOCHIZUKI, S. SAKURAI, K. β-1, 3-Glucan/antisense oligonucleotide complex stabilized with phosphorothioation and its gene suppression. Bioorg. Chem., 2010, vol. 38, pp. 260-264 see entire text | 1-40 |
| A | SANADA, Y. et al. β-1, 3-D-Glucan Schizophyllan/Poly(dA) Triple-Helical Complex in Dilute Solution. J. Phys. Chem. B., 2012, vol. 116, pp. 87-94 see entire text | 1-40 |
| A | 櫻井和朗, 多糖核酸複合体の発見と核酸医薬ＤＤＳへの応用, Drug Delivery Syst., 2018, vol. 33, no. 4, pp. 311-317 see entire text, (SAKURAI, Kazuo. Discovery of polysaccharide/polynucleotide complex and its application to DDS.) | 1-40 |
| A | 櫻井和朗, 分子分画法を利用した高分子複合体の特性評価, 高分子, 2018, vol. 67, no. 7, pp. 404-409 see entire text, (SAKURAI, Kazuo. Fractionation and Characterization of Macromolecular Complexes and Supermolecules. High polymers, Japan : polymers.) | 1-40 |
| A | 隅谷和樹ほか, 低分子量β-1,3-D-グルカンシゾフィラン／核酸複合体の構造解析, 日本核酸医薬学会第５回年会 講演要旨集, 2019, p.148 (P-068*) see entire text, (SUMIYA, Kazuki et al.), non-official translation (Structural analysis of low-molecular-weights β-1,3-D-glucan schizophyllan and nucleic acid complexing compounds. Lecture abstract of the 5th Annual Meeting of the Nucleic Acids Therapeutics Society of Japan.) | 1-40 |
| A | 隅谷和樹ほか, 低分子量β-1,3-D-グルカンシゾフィラン／核酸複合体の構造解析, 日本核酸医薬学会第４回年会　講演要旨集, 2018, p.132 (P-61*) see entire text, (SUMIYA, Kazuki et al.), non-official translation (Structural analysis of low-molecular-weights β-1,3-D-glucan schizophyllan and nucleic acid complexing compounds. Lecture abstract of the 4th Annual Meeting of the Nucleic Acids Therapeutics Society of Japan.) | 1-40 |

Form PCT/ISA/210 (second sheet) (January 2015)

**INTERNATIONAL SEARCH REPORT**

International application No.
**PCT/JP2021/037413**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/037413**

| Patent document cited in search report | Publication date (day/month/year) | Patent family member(s) | Publication date (day/month/year) |
|---|---|---|---|
| WO 2016/098832 A1 | 23 June 2016 | JP 2016-113404 A | |
| JP 2010-207109 A | 24 September 2010 | (Family: none) | |
| JP 2011-178707 A | 15 September 2011 | (Family: none) | |
| WO 2018/097296 A1 | 31 May 2018 | US 2019/0382762 A1<br>see entire text<br>EP 3546577 A1 | |
| JP 2008-100919 A | 01 May 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008100919 A
- WO 201504131 A
- JP 5605793 B
- JP 2020171532 A

### Non-patent literature cited in the description


- *Journal of the American Chemical Society,* 2000, vol. 122 (18), 4520-4521
- *Biomacromolecules,* 2001, vol. 2 (3), 641-650
- *J Am Chem Soc,* 2004, vol. 126 (27), 8372-3
- *Chem Commun (Camb),* 2005, vol. 35, 4383-98
- *Bioconjugate chemistry,* 2017, vol. 28 (2), 565-573
- *Journal of Controlled Release,* 2015, vol. 220, 495-502
- *Proceedings of the National Academy of Sciences,* 2014, vol. 111 (8), 3086-3091
- *International journal of molecular sciences,* 2020, vol. 21 (2), 683-693
- *Bioorganic Chemistry,* 2010, vol. 38 (6), 260-264
- *The Journal of Physical Chemistry,* 2012, vol. 116 (1), 87-94
- *Koubunshi,* 2018, vol. 67 (7), 404-409
- **SAKURAI et al.** *Biomacromolecules,* 2001, vol. 2, 641-650
- **SHIMADA et al.** *Bioconjugate chemistry,* 2007, vol. 18, 1280-1286
- **KOYAMA et al.** *Science translational medicine,* 2010, vol. 2, 25-24
- **MINARI et al.** *Bioconjugate chemistry,* 2011, vol. 22, 9-15
- *Cancer Gene Therapy,* 2019, vol. 26, 32-40